# EUROPEAN PATENT APPLICATION

(11) **EP 4 163 297 A1**
(43) Date of publication of application: **12.04.2023**
(21) Application number: 21831677.6
(22) Date of filing: 29.06.2021
(51) Int. Cl.: C07K 14/725, C12N 15/12, A61K 38/17

(54) **T CELL ANTIGEN RECEPTOR, MULTIMERIC COMPLEX THEREOF AND PREPARATION METHOD AND USE THEREOF**

(30) Priority: 01.07.2020 CN 202010632672
(71) Applicant: Bristar Immunotech Limited, Beijing 102200 (CN); Tsinghua University, Haidian District Beijing 100084 (CN)
(72) Inventor: CHEN, Hua, Beijing 102200 (CN); LIU, Guangna, Beijing 102200 (CN); LEI, Lei, Beijing 102200 (CN); LIU, Fang, Beijing 102200 (CN); JIA, Lemei, Beijing 102200 (CN); HUANG, Daosheng, Beijing 102200 (CN); ZHAO, Xueqiang, Beijing 102200 (CN); LIN, Xin, Beijing 102200 (CN)
(74) Representative: HGF
(86) International application number: PCT/CN2021/102954
(87) International publication number: WO 2022/002014

(57) **Abstract**

A T cell antigen receptor, an immune cell for expressing the T cell antigen receptor (TCR) and a preparation method and use thereof. The TCR disclosed in the present invention can be specifically activated by virus antigen peptide presenting cells, so that the release level of extracellular cytokines IFNγ and IL2 and the release amount of lactate dehydrogenase are improved, and target cells are significantly killed.

## Description

### TECHNICAL FIELD

The present invention relates to the technical field of T cell antigen receptors capable of recognizing CMV pp65, and in particular to a plurality of TCRs and use thereof in treating and/or preventing CMV-related diseases.

### BACKGROUND

The T cell antigen receptor (T cell receptor, TCR) is crucial for the cellular immune function of the immune system, and the TCR is the only receptor for specific antigen peptides presented on major histocompatibility complexes (MHCs), and the combination of the antigen specific TCR and the pMHC complex triggers the direct physical contact and interaction of T cells and antigen presenting cells, leading to a series of subsequent cell signaling and other physiological reactions, thereby enabling the T cells with different antigen specificities to exert immune effects on target cells thereof.

CN107827959A discloses a method for isolating antigen-specific T cells and cloning the gene encoding the antigen-specific TCR in the antigen-specific T cells. The TCR can be combined with HBVS183-91 antigen short-peptide complex FLLTRILTI-HLA*A0201, and meanwhile, cells transduced with the TCR of the present invention can be specifically activated and have a strong killing effect on target cells. CN106279404A discloses a soluble and stable heterodimeric TCR comprising an artificial interchain disulfide bond between the α chain variable region and the β chain constant region, wherein the TCR is soluble and stable and can be well renatured, refolded and purified and can simultaneously specifically bind to the original ligand. None of the above patents disclose a specific TCR against cytomegalovirus.

Cytomegalovirus (CMV) is a herpesvirus DNA virus, also known as a cell inclusion body virus. The cytomegalovirus is widely distributed and people are generally susceptible to the virus, with the infection rate reaching 95% or more in Chinese adults. Generally, the infected people are detected negative, with most infected people having no clinical symptoms, but they may have inflammatory diseases after the virus attacks a plurality of organs and systems under certain conditions. CN102656188A discloses a T cell receptor capable of recognizing an antigen from cytomegalovirus, wherein the T cell receptor, when presented on the major histocompatibility complex (MHC), specifically binds to a peptide from the cytomegalovirus (CMV) phosphoprotein pp65, the peptide having the amino acid sequence NLVPMVATV However, the killing effect of the T cell receptor on the target cells is not obvious in the application.

Therefore, the present invention provides a T cell antigen receptor capable of being specifically activated by virus antigen peptide presenting cells, so that the release level of the extracellular cytokines IFNγ and IL2 and the release amount of lactate dehydrogenase are improved, and target cells are significantly killed.

### SUMMARY

The present invention provides a plurality of T cell antigen receptors (TCRs) specifically binding to antigen peptides from the cytomegalovirus (CMV) phosphoprotein pp65 that are presented on MHC molecules, wherein the CMV phosphoprotein pp65 has an amino acid sequence comprising SEQ ID NO: 34. The TCRs of the present invention can specifically recognize corresponding CMV pp65 antigen peptide-MHC molecule complexes, activate TCR T cells, and further generate high-level cytokines IFNγ, IL2 and TNFα, so that tumor cells are significantly killed in an *in vivo* or *in vitro* test. The present invention is specifically as follows:
In a first aspect of the present invention, provided is a complementarity determining region (CDR) binding to CMV pp65, wherein the CDR is selected from one of or a combination of two or more of SEQ ID NOs: 4-33. Preferably, the CDR comprises CDR1α-CDR3α and/or CDR1β-CDR3β, wherein the CDR1α has an amino acid sequence comprising any one of SEQ ID NOs: 4-7 or having at least 80% homology to any one of SEQ ID NOs: 4-7, the CDR2α has an amino acid sequence comprising any one of SEQ ID NOs: 8-11 or having at least 80% homology to any one of SEQ ID NOs: 8-11, the CDR3α has an amino acid sequence comprising any one of SEQ ID NOs: 12-17 or having at least 80% homology to any one of SEQ ID NOs: 12-17, the CDR1β has an amino acid sequence comprising any one of SEQ ID NOs: 18-22 or having at least 80% homology to any one of SEQ ID NOs: 18-22, the CDR2β has an amino acid sequence comprising any one of SEQ ID NOs: 23-27 or having at least 80% homology to any one of SEQ ID NOs: 23-27, and the CDR3β has an amino acid sequence comprising any one of SEQ ID NOs: 28-33 or having at least 80% homology to any one of SEQ ID NOs: 28-33.

In a specific embodiment of the present invention, the CDR is selected from any one of the following groups:

| CDR1α | CDR2α | CDR3α | CDR1β | CDR2β | CDR3β |
|---|---|---|---|---|---|
| SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |
| SSNFYA (SEQ ID NO: 4) | MTLNGDE (SEQ ID NO: 8) | ASGTYKYI (SEQ ID NO: 13) | MNHEY (SEQ ID NO: 18) | SVGAGI (SEQ ID NO: 23) | ASRPLGVGETQY (SEQ ID NO: 29) |
| NSAFQY (SEQ ID NO: 5) | TYSSGN (SEQ ID NO: 9) | ATFLTGNQFY (SEQ ID NO: 14) | SGHDY (SEQ ID NO: 19) | FNNNVP (SEQ ID NO: 24) | ASSFSLPYEQY (SEQ ID NO: 30) |
| SSNFYA (SEQ ID NO: 4) | MTLNGDE (SEQ ID NO: 8) | ARYGNKLV (SEQ ID NO: 15) | SGHVS (SEQ ID NO: 20) | FQNEAQ (SEQ ID NO: 25) | ASSFYTGQETQY (SEQ ID NO: 31) |
| TSESDYY (SEQ ID NO: 6) | QEAYKQQN (SEQ ID NO: 10) | AYRAFYTGANSKLT (SEQ ID NO: 16) | SGHDT (SEQ ID NO: 21) | YYEEEE (SEQ ID NO: 26) | ASSFFRGEGNQPQH (SEQ ID NO: 32) |
| NSMFDY (SEQ ID NO: 7) | ISSIKDK (SEQ ID NO: 11) | AASAWNTGNQFY (SEQ ID NO: 17) | SGHRS (SEQ ID NO: 22) | YFSETQ (SEQ ID NO: 27) | ASSLAGYKQETQY (SEQ ID NO: 33) |

In a second aspect of the present invention, provided is an α-chain polypeptide binding to CMV pp65, wherein the α-chain polypeptide comprises a CDR1α, a CDR2α and/or a CDR3α, wherein the CDR1α has an amino acid sequence comprising any one of SEQ ID NOs: 4-7 or having at least 80% homology to any one of SEQ ID NOs: 4-7, the CDR2α has an amino acid sequence comprising any one of SEQ ID NOs: 8-11 or having at least 80% homology to any one of SEQ ID NOs: 8-11, and the CDR3α has an amino acid sequence comprising any one of SEQ ID NOs: 12-17 or having at least 80% homology to any one of SEQ ID NOs: 12-17.

In a specific embodiment of the present invention, the α-chain polypeptide comprises CDR1α-CDR3α of any one of the following groups:

| CDR1α | CDR2α | CDR3α |
|---|---|---|
| SSNFYA (SEQ ID NO: 4) | MTLNGDE (SEQ ID NO: 8) | ARNTGNQFY (SEQ ID NO: 12) |
| SSNFYA (SEQ ID NO: 4) | MTLNGDE (SEQ ID NO: 8) | ASGTYKYI (SEQ ID NO: 13) |
| NSAFQY (SEQ ID NO: 5) | TYSSGN (SEQ ID NO: 9) | ATFLTGNQFY (SEQ ID NO: 14) |
| SSNFYA (SEQ ID NO: 4) | MTLNGDE (SEQ ID NO: 8) | ARYGNKLV (SEQ ID NO: 15) |
| TSESDYY (SEQ ID NO: 6) | QEAYKQQN (SEQ ID NO: 10) | AYRAFYTGANSKLT (SEQ ID NO: 16) |
| NSMFDY (SEQ ID NO: 7) | ISSIKDK (SEQ ID NO: 11) | AASAWNTGNQFY (SEQ ID NO: 17) |

In a third aspect of the present invention, provided is a β-chain polypeptide binding to CMV pp65, wherein the β-chain polypeptide comprises a CDR1β, a CDR2β and/or a CDR3β, wherein the CDR1β has an amino acid sequence comprising any one of SEQ ID NOs: 18-22 or having at least 80% homology to any one of SEQ ID NOs: 18-22, the CDR2β has an amino acid sequence comprising any one of SEQ ID NOs: 23-27 or having at least 80% homology to any one of SEQ ID NOs: 23-27, and the CDR3β has an amino acid sequence comprising any one of SEQ ID NOs: 28-33 or having at least 80% homology to any one of SEQ ID NOs: 28-33.

Preferably, the β-chain polypeptide comprises CDR1β-CDR3β of any one of the following groups:

| CDR1β | CDR2β | CDR3β |
|---|---|---|
| MNHEY (SEQ ID NO: 18) | SVGAGI (SEQ ID NO: 23) | ASSFATGVGSYGYT (SEQ ID NO: 28) |
| MNHEY (SEQ ID NO: 18) | SVGAGI (SEQ ID NO: 23) | ASRPLGVGETQY (SEQ ID NO: 29) |
| SGHDY (SEQ ID NO: 19) | FNNNVP (SEQ ID NO: 24) | ASSFSLPYEQY (SEQ ID NO: 30) |
| SGHVS (SEQ ID NO: 20) | FQNEAQ (SEQ ID NO: 25) | ASSFYTGQETQY (SEQ ID NO: 31) |
| SGHDT (SEQ ID NO: 21) | YYEEEE (SEQ ID NO: 26) | ASSFFRGEGNQPQH (SEQ ID NO: 32) |
| SGHRS (SEQ ID NO: 22) | YFSETQ (SEQ ID NO: 27) | ASSLAGYKQETQY (SEQ ID NO: 33) |

In a fourth aspect of the present invention, provided is a T cell antigen receptor specifically binding to CMV pp65.

Preferably, the CMV pp65 has a binding epitope comprising any one of or a combination of two or three of SEQ ID NOs: 1-3.

Preferably, the T cell antigen receptor specifically binds to an antigen peptide from the cytomegalovirus (CMV) phosphoprotein pp65 by presentation on the major histocompatibility complex (MHC).

Preferably, the T cell antigen receptor comprises at least one α-chain variable region and/or β-chain variable region.

Preferably, the T cell antigen receptor is an α-β heterodimer.

Preferably, the T cell antigen receptor comprises α-chain CDR1α-CDR3α and β-chain CDR1β-CDR3β.

More preferably, the CDR3α has an amino acid sequence comprising any one of SEQ ID NOs: 12-17 or having at least 80% homology to any one of SEQ ID NOs: 12-17.

More preferably, the CDR3β has an amino acid sequence comprising any one of SEQ ID NOs: 28-33 or having at least 80% homology to any one of SEQ ID NOs: 28-33.

More preferably, the CDR1α has an amino acid sequence comprising any one of SEQ ID NOs: 4-7 or having at least 80% homology to any one of SEQ ID NOs: 4-7.

More preferably, the CDR2α has an amino acid sequence comprising any one of SEQ ID NOs: 8-11 or having at least 80% homology to any one of SEQ ID NOs: 8-11.

More preferably, the CDR1β has an amino acid sequence comprising any one of SEQ ID NOs: 18-22 or having at least 80% homology to any one of SEQ ID NOs: 18-22.

More preferably, the CDR2β has an amino acid sequence comprising any one of SEQ ID NOs: 23-27 or having at least 80% homology to any one of SEQ ID NOs: 23-27.

Even more preferably, the CDR1α-CDR3α and CDR1β-CDR3β comprise any one of the following groups:

| TCR | Binding epitope | CDR1α | CDR2α | CDR3α | CDR1β | CDR2β | CDR3β |
|---|---|---|---|---|---|---|---|
| C22 | SEQ ID NO: 1 | SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |
| C27 | SEQ ID NO: 2 | SSNFYA (SEQ ID NO: 4) | MTLNGDE (SEQ ID NO: 8) | ASGTYKYI (SEQ ID NO: 13) | MNHEY (SEQ ID NO: 18) | SVGAGI (SEQ ID NO: 23) | ASRPLGVGETQY (SEQ ID NO: 29) |
| C29 | SEQ ID NO: 1 | NSAFQY(SEQ ID NO: 5) | TYSSGN (SEQ ID NO: 9) | ATFLTGNQFY (SEQ ID NO: 14) | SGHDY (SEQ ID NO: 19) | FNNNVP (SEQ ID NO: 24) | ASSFSLPYEQY(SEQ ID NO: 30) |
| C30 | SEQ ID NO: 2 | SSNFYA(SEQ ID NO: 4) | MTLNGDE (SEQ ID NO: 8) | ARYGNKLV (SEQ ID NO: 15) | SGHVS (SEQ ID NO: 20) | FQNEAQ (SEQ ID NO: 25) | ASSFYTGQETQY (SEQ ID NO: 31) |
| C44 | SEQ ID NO: 3 | TSESDYY (SEQ ID NO: 6) | QEAYKQQN (SEQ ID NO: 10) | AYRAFYTGANSKLT (SEQ ID NO: 16) | SGHDT (SEQ ID NO: 21) | YYEEEE (SEQ ID NO: 26) | ASSFFRGEGNQPQH (SEQ ID NO: 32) |
| C45 | SEQ ID NO: 3 | NSMFDY (SEQ ID NO: 7) | ISSIKDK (SEQ ID NO: 11) | AASAWNTGNQFY (SEQ ID NO: 17) | SGHRS (SEQ ID NO: 22) | YFSETQ (SEQ ID NO: 27) | ASSLAGYKQETQY (SEQ ID NO: 33) |

In a specific embodiment of the present invention, the β chain has an amino acid sequence comprising any one of SEQ ID NOs: 247-252 or having at least 80% homology to any one of SEQ ID NOs: 247-252.

In a specific embodiment of the present invention, the α chain has an amino acid sequence comprising any one of SEQ ID NOs: 253-258 or having at least 80% homology to any one of SEQ ID NOs: 253-258.

Preferably, amino acids of the α chain and the β chain are linked directly or indirectly, preferably linked indirectly, and more preferably via fp2A (furin-SGSG-p2A).

More preferably, the fp2A has an amino acid sequence comprising SEQ ID NO: 259.

In a specific embodiment of the present invention, the α chain and the β chain may be linked by the following order: the α chain, fp2A and the β chain, or, the β chain, fp2A and the α chain.

In a specific embodiment of the present invention, the T cell antigen receptor has an amino acid sequence comprising any one of SEQ ID NOs: 36, 38, 40, 42, 44 or 46 or having at least 80% homology to any one of SEQ ID NOs: 36, 38, 40, 42, 44 or 46.

In a fifth aspect of the present invention, provided is an antibody or an antigen-binding fragment thereof specifically binding to CMV pp65.

Preferably, the CMV pp65 has a binding epitope comprising any one of or a combination of two or three of SEQ ID NOs: 1-3.

Preferably, the antibody or the antigen-binding fragment thereof comprises CDR H1-CDR H3 of a heavy chain and/or CDR L1-CDR L3 of a light chain.

Still more preferably, the light chain of the antibody or the antigen-binding fragment thereof has an amino acid sequence comprising any one of SEQ ID NOs: 247-252 or having at least 80% homology to any one of SEQ ID NOs: 247-252.

Still more preferably, the heavy chain of the antibody or the antigen-binding fragment thereof has an amino acid sequence comprising any one of SEQ ID NOs: 253-258 or having at least 80% homology to any one of SEQ ID NOs: 253-258.

Preferably, the antibody or the antigen-binding fragment thereof is a single domain antibody or a single chain antibody scFv.

Preferably, amino acids of the heavy chain and the light chain of the antibody or the antigen-binding fragment thereof are linked directly or indirectly, preferably linked indirectly, and more preferably via p2A.

More preferably, the p2A is fp2A having an amino acid sequence comprising SEQ ID NO: 259.

In a specific embodiment of the present invention, the heavy chain and the light chain may be linked by the following order: the heavy chain, fp2A and the light chain, or, the light chain, fp2A and the heavy chain. Preferably, the antibody or the antigen-binding fragment thereof may further comprise a fragment such as a Fab, Fab', Fab'-SH, Fv, scFv, (Fab')₂, a single domain antibody, a diabody (dAb), or a linear antibody.

In a specific embodiment of the present invention, the antibody or the antigen-binding fragment thereof has an amino acid sequence comprising any one of SEQ ID NOs: 36, 38, 40, 42, 44 or 46 or having at least 80% homology to any one of SEQ ID NOs: 36, 38, 40, 42, 44 or 46.

In a sixth aspect of the present invention, provided is a nucleic acid encoding the CDR described above.

In a seventh aspect of the present invention, provided is a nucleic acid encoding the β chain of the T cell antigen receptor of the present invention or the β-chain polypeptide described above.

Preferably, the nucleic acid encoding the β chain of the T cell antigen receptor has a sequence comprising any one of SEQ ID NOs: 260-265 or having at least 80% homology to any one of SEQ ID NOs: 260-265.

In an eighth aspect of the present invention, provided is a nucleic acid encoding the α chain of the T cell antigen receptor of the present invention or the α-chain polypeptide described above.

Preferably, the nucleic acid encoding the α chain of the T cell antigen receptor has a sequence comprising any one of SEQ ID NOs: 266-271 or having at least 80% homology to any one of SEQ ID NOs: 266-271.

In a ninth aspect of the present invention, provided is a nucleic acid encoding the T cell antigen receptor or the antibody or the antigen-binding fragment thereof of the present invention.

Preferably, the nucleic acid encoding the α chain and the nucleic acid encoding the β chain of the T cell antigen receptor are linked via a nucleic acid sequence encoding fp2A. More preferably, the nucleic acid encoding fp2A has a sequence comprising SEQ ID NO: 272.

Preferably, the nucleic acid encoding the T cell antigen receptor has a sequence comprising any one of SEQ ID NOs: 37, 39, 41, 43, 45 or 47 or having at least 80% homology to any one of SEQ ID NOs: 37, 39, 41, 43, 45 or 47.

Preferably, the nucleic acid encoding the antibody or the antigen-binding fragment thereof has a sequence comprising any one of SEQ ID NOs: 37, 39, 41, 43, 45 or 47 or having at least 80% homology to any one of SEQ ID NOs: 37, 39, 41, 43, 45 or 47.

The nucleic acid sequence of the present invention may be single-stranded or double-stranded, and may be DNA or RNA.

Preferably, the nucleic acid sequence may be codon-optimized. More preferably, the codon optimization comprises changing a number of rare codons used by a virus or the like into corresponding mammalian codons, and/or removing mRNA unstable motifs and/or cryptic splicing sites.

In a tenth aspect of the present invention, provided is an expression vector comprising the nucleic acid according to any embodiment of the present invention.

Preferably, the expression vector can express *in vivo* or *in vitro* or *ex vivo.* More preferably, the expression vector expresses at a high level continuously in a cell *in vivo.*

Preferably, the expression vector may be a prokaryotic expression vector or a retroviral vector.

More preferably, the prokaryotic expression vector is *Escherichia coli* series. In a specific embodiment of the present invention, the expression vector is pET-26b or pET28a+.

More preferably, the virus may be Rous sarcoma virus (RSV), lentivirus, human immunodeficiency virus (HIV), murine leukemia virus (MLV), equine infectious anemia virus (EIAV), mouse mammary tumor virus (MMTV), Fujinami sarcoma virus (FuSV), FBR murine sarcoma virus (FBR MSV), Moloney murine leukemia virus (Mo-MLV), Moloney murine sarcoma virus (Mo-MSV), Abelson murine leukemia virus (A-MLV), avian myelocytomatosis virus 29 (MC29), avian erythroblastosis virus (AEV), or the like. Even more preferably, the expression vector is a lentiviral expression vector.

In a specific embodiment of the present invention, the expression vector is pHAGE-IRES-RFP.

More preferably, the β chain, the α chain and the vector backbone in the expression vector are linked by the following order: a promoter, the β chain, fp2A, the α chain, IRES and RFP sequences.

In an eleventh aspect of the present invention, provided is a host cell comprising the nucleic acid or the expression vector according to any embodiment of the present invention.

Preferably, the host cell may be eukaryotic or prokaryotic. More preferably, the host cell is a yeast cell, a 293 cell, a CHO cell, *Escherichia coli,* or the like.

In a specific embodiment of the present invention, the host cell is Stbl3, BL21 or transetta.

In a twelfth aspect of the present invention, provided is an immune cell expressing the CDR, the α-chain polypeptide, the β-chain polypeptide, the T cell antigen receptor or the antibody or the antigen-binding fragment thereof of the present invention.

Preferably, the immune cell comprises one or more of the heterologous nucleic acid sequences according to any embodiment of the present invention.

Preferably, the immune cell includes, but is not limited to, lymphocytes (including T cells and B cells). Furthermore, the immune cell is a B cell expressing the antibody or the antigen-binding fragment thereof described above. The immune cell is a T cell having a T cell antigen receptor structure as defined above. Preferably, the T cell may be CD4+ T cells, CD8+ T cells, etc.

More preferably, the immune cell is isolated from a T cell derived from a subject.

Preferably, the immune cell is a T cell from a CMV seronegative donor.

In a thirteenth aspect of the present invention, provided is a method for preparing an immune cell, which comprises transfecting an immune cell with a nucleic acid sequence encoding the CDR, the α-chain polypeptide, the β-chain polypeptide, the antibody or the antigen-binding fragment thereof, or the T cell antigen receptor as described above for expression.

Preferably, the immune cell includes, but is not limited to, stem cells, lymphocytes (including T cells and B cells). Furthermore, the immune cell is a B cell expressing the antibody or the antigen-binding fragment thereof described above. The immune cell is a T cell having a T cell antigen receptor structure as defined above. Preferably, the method further comprises a step of knocking out an endogenous TCR of the cell. Specifically, a guide targeting the endogenous TCR can be constructed into a lentiviral vector, and co-transfected with a packaging plasmid and a transfection reagent into a T cell.

In a fourteenth aspect of the present invention, provided is a method for preparing a recombinant T cell, which comprises the following steps:
1) obtaining the nucleic acid according to any embodiment of the present invention from a positive T cell clone;
2) separating, culturing a primary T cell; and
3) delivering the nucleic acid obtained in the step 1) to the primary T cell in the step 2) to obtain a recombinant T cell expressing the CDR, the α-chain polypeptide, the β-chain polypeptide, the antibody or the antigen-binding fragment thereof or the T cell antigen receptor according to any embodiment of the present invention. Preferably, the T cell is selected from hematopoietic stem cells or peripheral blood lymphocyte (PBL)-derived T cells.

In a fifteenth aspect of the present invention, provided is a method for preparing an antibody or an antigen-binding fragment thereof or a T cell antigen receptor, which comprises the following steps:
(1) obtaining the nucleic acid according to any embodiment of the present invention from a positive T cell clone;
(2) connecting the nucleic acid obtained in the step (1) to a vector backbone to obtain an expression vector;
(3) transforming the expression vector obtained in the step (2) into a host cell, and then inducing the expression of the host cell;
(4) obtaining the antibody or the antigen-binding fragment thereof or the T cell antigen receptor.

Preferably, the positive T cell specifically binds to an antigen peptide from MHC-presented cytomegalovirus (CMV) phosphoprotein pp65. More preferably, the MHC-presented cytomegalovirus (CMV) phosphoprotein pp65 antigen peptide complex is a monomer or multimeric complex.

In a sixteenth aspect of the present invention, provided is a multimeric complex comprising the T cell antigen receptor according to any embodiment of the present invention.

Preferably, the multimeric complex further comprises a monomer, a biotin molecule, and a streptavidin or avidin molecule, wherein the monomer comprises an α-chain extracellular domain of an MHC molecule, a β2m chain and an antigen peptide, and the monomer is conjugated to the biotin molecule binding to the streptavidin or avidin molecule.

Preferably, the α-chain extracellular domain of the MHC molecule is connected with an avi-tag sequence at the C-terminus.

Preferably, the α-chain extracellular domain of the MHC molecule does not contain a signal peptide sequence, with an amino acid M added before a mature peptide sequence.

Preferably, the β2m chain does not contain a signal peptide sequence, with two amino acids M and A added before a mature peptide sequence.

In a specific embodiment of the present invention, the β2m chain does not contain a signal peptide, with two amino acids, preferably M and A, added before a mature peptide sequence.

Preferably, the antigen peptide comprises any one of or a combination of two or more of SEQ ID NOs: 1-3.

In a specific embodiment of the present invention, the multimeric complex comprises:
(1) a T cell antigen receptor, preferably, the T cell antigen receptor has an amino acid sequence comprising any one of SEQ ID NOs: 36, 38, 40, 42, 44 or 46 or having at least 80% homology to any one of SEQ ID NOs: 36, 38, 40, 42, 44 or 46.
(3) a monomer, comprising an antigen peptide, an α-chain extracellular domain of an MHC molecule connected with an avi-tag sequence at the C terminus and a β2m chain without a signal peptide; wherein the antigen peptide is selected from any one of or a combination of two or more of SEQ ID NOs: 1-3;
(4) a biotin molecule; and
(5) a streptavidin molecule or an avidin molecule; wherein the monomer is conjugated to the biotin molecule binding to the streptavidin or avidin.

Preferably, the MHC molecule is an MHC class I molecule or an MHC class II molecule. More preferably, the MHC molecule is an MHC class I molecule.

Preferably, the MHC molecule is selected from HLA-A*0201, HLA-A*2402 and HLA-A*1101.

In a specific embodiment of the present invention, the α chain of the MHC molecule has an amino acid sequence set forth in any one of SEQ ID NOs: 48, 50 or 52 or having at least 80% homology to any one of SEQ ID NOs: 48, 50 or 52.

In a specific embodiment of the present invention, the α chain of the MHC molecule has a nucleotide sequence set forth in any one of SEQ ID NOs: 49, 51 or 53 or having at least 80% homology to any one of SEQ ID NOs: 49, 51 or 53.

In a specific embodiment of the present invention, the β2m chain of the MHC molecule has an amino acid sequence set forth in SEQ ID NO: 54 or having at least 80% homology to SEQ ID NO: 54.

In a specific embodiment of the present invention, the β2m chain of the MHC molecule has a nucleotide sequence set forth in SEQ ID NO: 55 or having at least 80% homology to SEQ ID NO: 55.

To increase the specificity of binding of an antigen peptide-MHC tetramer to a T cell antigen receptor, the monomer further comprises a chemical modification, mutation, insertion and/or deletion of at least one amino acid.

Preferably, the α-chain extracellular domain of the MHC molecule and the β2m chain are non-covalently bound. Preferably, the multimeric complex comprises at least one monomer.

Preferably, each monomer is conjugated to at least one biotin molecule.

In a seventeenth aspect of the present invention, provided is a method for preparing the multimeric complex according to any embodiment of the present invention, which comprises the following steps:
I) expressing and purifying an α-chain extracellular domain of an MHC molecule connected with an avi-tag sequence at the C terminus and a β2m chain;
II) refolding an antigen peptide, the β2m chain obtained in the step 1), and the α-chain extracellular domain of the MHC molecule connected with the avi-tag sequence at the C terminus to prepare a monomer;
III) biotinylating the monomer prepared in the step II) to obtain a biotinylated monomer;
IV) subjecting the biotinylated monomer obtained in the step III) to a reaction with fluorescently labeled streptavidin or avidin to prepare an antigen peptide-MHC molecule tetramer;
V) co-incubating the antigen peptide-MHC molecule tetramer obtained in the step IV) with T cells to form a complex of a T cell antigen receptor and the antigen peptide-MHC molecule tetramer.

Preferably, the step I) comprises separately cloning a nucleotide sequence encoding the α-chain extracellular domain of the MHC molecule connected with the avi-tag sequence at the C-terminus and a nucleotide sequence encoding the β2m chain of the MHC molecule, connecting the cloned sequences to a vector, transforming the vector into an expression strain, culturing the expression strain, adding an inducer, and extracting inclusion bodies.

More preferably, the expression strain is cultured until the OD₆₀₀ value is between 0.2 and 0.4.

More preferably, the final molar concentration of the inducer after addition is between 0.5 mM and 1 mM. Preferably, the expression is induced for 4-6 h.

Preferably, the step II) comprises refolding of the β2m chain, that is, sequentially adding the antigen peptide, the β2m chain of the MHC molecule, and the α-chain extracellular domain of the MHC molecule connected with the avi-tag sequence at the C-terminus into a dilution buffer for a water bath away from light, wherein the refolding of the β2m chain includes denaturation of inclusion bodies, addition of a protease inhibitor, and then dialysis. Preferably, the antigen peptide, the β2m chain without the signal peptide and the α chain connected with the avi-tag sequence at the C-terminus are at a molar ratio of (30-50):(2-2.5): 1, more preferably 40:2:1.

Preferably, the step II) further comprises a step of purifying the monomer.

Preferably, the biotinylation in the step III) is performed by binding the monomer to BiomixA or BiomixB under the catalysis of BirA enzyme.

Preferably, the step III) further comprises a step of purifying the biotinylated monomer.

Preferably, in the step IV), the monomer is reacted with streptavidin at a molar ratio of (4-7): 1.

In an eighteenth aspect of the present invention, provided is use of the multimeric complex described above in preparing, screening or detecting the antibody or the antigen-binding fragment thereof or the T cell antigen receptor of the present invention.

In a nineteenth aspect of the present invention, provided is a method for preparing a T cell antigen receptor, which comprises the following steps:
I) expressing and purifying an α-chain extracellular domain of an MHC molecule connected with an avi-tag sequence at the C terminus and a β2m chain;
II) refolding an antigen peptide, the α-chain extracellular domain of the MHC molecule connected with the avi-tag sequence at the C terminus and the β2m chain obtained in the step I) to prepare a monomer;
III) biotinylating the monomer prepared in the step II) to obtain a biotinylated monomer;
IV) subjecting the biotinylated monomer obtained in the step III) to a reaction with fluorescently labeled streptavidin or avidin to prepare an antigen peptide-MHC molecule tetramer; and
V) co-incubating the antigen peptide-MHC molecule tetramer obtained in the step IV) with T cells to form a complex of a T cell antigen receptor and the antigen peptide-MHC molecule tetramer for isolation to obtain a T cell antigen receptor.

In a twentieth aspect of the present invention, provided is use of the CDR according to any embodiment of the present invention, the α-chain polypeptide according to any embodiment of the present invention, the β-chain polypeptide according to any embodiment of the present invention, the T cell antigen receptor according to any embodiment of the present invention, the antibody or the antigen-binding fragment thereof according to any embodiment of the present invention, the nucleic acid according to any embodiment of the present invention, the expression vector according to any embodiment of the present invention, the host cell according to any embodiment of the present invention, the immune cell according to any embodiment of the present invention, or the multimeric complex according to any embodiment of the present invention in preparing a product for the diagnosis or treatment of a tumor or a CMV-related disease.

Preferably, the CMV-related disease is selected from neonatal CMV inclusion body disease, acute acquired CMV infection or diseases caused by CMV infection of an immunocompromised person.

In a specific embodiment of the present invention, the CMV-related disease is selected from liver, spleen or central nervous system disease, disability, infectious mononucleosis, skeletal muscle pain, CMV retinitis, gastrointestinal CMV, or encephalitis, and the like.

In a twenty-first aspect of the present invention, provided is use of the CDR according to any embodiment of the present invention, the α-chain polypeptide according to any embodiment of the present invention, the β-chain polypeptide according to any embodiment of the present invention, the T cell antigen receptor according to any embodiment of the present invention, the antibody or the antigen-binding fragment thereof according to any embodiment of the present invention, the nucleic acid according to any embodiment of the present invention, the expression vector according to any embodiment of the present invention, the host cell according to any embodiment of the present invention, the immune cell according to any embodiment of the present invention, or the multimeric complex according to any embodiment of the present invention in labeling, detection, cell sorting, or activation of T cells.

In a twenty-second aspect of the present invention, provided is a pharmaceutical composition comprising any one of the following groups:
i) the CDR according to any embodiment of the present invention;
ii) the α-chain polypeptide according to any embodiment of the present invention;
iii) the β-chain polypeptide according to any embodiment of the present invention;
iv) the antibody or the antigen-binding fragment thereof according to any embodiment of the present invention;
v) the T cell antigen receptor according to any embodiment of the present invention;
vi) the nucleic acid according to any embodiment of the present invention;
vii) the expression vector according to any embodiment of the present invention;
viii) the host cell according to any embodiment of the present invention;
ix) the immune cell according to any embodiment of the present invention; or
x) the multimeric complex according to any embodiment of the present invention.

Preferably, the pharmaceutical composition further comprises a pharmaceutically acceptable excipient.

Preferably, the pharmaceutical composition may also be used in combination with an additional therapeutic agent.

More preferably, the therapeutic agent may be an immunomodulator.

In a twenty-third aspect of the present invention, provided is a kit comprising any one of the following groups:
i) the CDR according to any embodiment of the present invention;
ii) the α-chain polypeptide according to any embodiment of the present invention;
iii) the β-chain polypeptide according to any embodiment of the present invention;
iv) the antibody or the antigen-binding fragment thereof according to any embodiment of the present invention;
v) the T cell antigen receptor according to any embodiment of the present invention;
vi) the nucleic acid according to any embodiment of the present invention;
vii) the expression vector according to any embodiment of the present invention;
viii) the host cell according to any embodiment of the present invention;
ix) the immune cell according to any embodiment of the present invention; or
x) the multimeric complex according to any embodiment of the present invention.

In a twenty-fourth aspect of the present invention, provided is a method for detecting cytomegalovirus (CMV) phosphoprotein pp65, which comprises making a sample to be tested in contact with the antibody or the antigen-binding fragment thereof of the present invention or the T cell antigen receptor of the present invention, and detecting a complex formed between the CMV phosphoprotein pp65 and the antibody or the antigen-binding fragment thereof or the T cell antigen receptor.

Preferably, the detection of the cytomegalovirus (CMV) phosphoprotein pp65 is to detect the presence or content of the cytomegalovirus (CMV) phosphoprotein pp65. The presence indicates presence or absence, and the content may be an expression level, a protein concentration, or the like.

Preferably, the antibody or the antigen-binding fragment thereof or the T cell antigen receptor comprises a detectable marker.

In a specific embodiment of the present invention, the marker may be His and/or HA.

The method for detecting cytomegalovirus (CMV) phosphoprotein pp65 of the present invention is not a method for diagnosing diseases. Firstly, the sample to be tested is not an organism or an *ex vivo* tissue or cell thereof, and secondly, even if cytomegalovirus (CMV) phosphoprotein pp65 is present in an organism or an organism comprises a certain concentration or expression level of CMV pp65, it cannot be determined that there is a disease, but only a possibility.

In a twenty-fifth aspect of the present invention, provided is a method for treating and/or preventing a CMV-related disease, which comprises administering to an individual an effective amount of the antibody or the antigen-binding fragment thereof, the T cell antigen receptor, the nucleic acid, the expression vector, the host cell, the immune cell or the pharmaceutical composition of the present invention.

Preferably, the method comprises a step of adoptively transferring T cells expressing the T cell antigen receptor of the present invention to a subject.

Preferably, the method comprises localizing the T cell antigen receptor of the present invention in the vicinity of the CMV-related disease (preferably a tumor or metastatic tumor) to increase the efficacy of a toxin or an immunostimulant.

Preferably, the method is used for the treatment and/or prevention of CMV reactivation following allogeneic hematopoietic stem cell transplantation.

Preferably, the method is used for the treatment and/or prevention of CMV reactivation following organ transplantation (e.g., kidney, liver, pancreas, intestine, cornea), tissue transplantation, cell transplantation (islet cells, limbal stem cells) or stem cell therapy.

Preferably, the T cells expressing the T cell antigen receptor of the present invention are derived from a subject. Preferably, the T cells expressing the T cell antigen receptor of the present invention are derived from the same donor as the hematopoietic stem cell, organ, tissue, cells or stem cells.

In a twenty-sixth aspect of the present invention, provided is a method for diagnosing a CMV-related disease, which comprises sampling, making a sample in contact with the antibody or the antigen-binding fragment thereof or the T cell antigen receptor of the present invention, and then detecting a complex formed by CMV pp65 and the antibody or the antigen-binding fragment thereof or the T cell antigen receptor.

Preferably, the antibody or the antigen-binding fragment thereof or the T cell antigen receptor comprises a detectable marker.

The TCRs of the present invention can specifically recognize corresponding CMV pp65 antigen peptide-MHC molecule complexes, activate TCR T cells, and further generate high-level cytokines IFNγ, IL2 and TNFα, so that tumor cells are significantly killed in an *in vivo* or *in vitro* test.

"T cell antigen receptor" described herein is a molecule capable of recognizing a peptide when presented by an MHC molecule or a tetramer thereof.

"Tumor" described herein includes, but is not limited to, pancreatic cancer, liver cancer, colon cancer, rectal cancer, stomach cancer, lymphoma, basal cell carcinoma, non-small cell lung cancer, leukemia, ovarian cancer, nasopharyngeal cancer, breast cancer, endometrial cancer, bladder cancer, lung cancer, bronchial cancer, bone cancer, prostate cancer, bile duct cancer, esophageal cancer, kidney cancer, thyroid cancer, head and neck cancer, testicular cancer, glioblastoma, astrocytoma, melanoma, myelodysplastic syndrome, and sarcoma, wherein the leukemia is selected from acute lymphocytic (lymphoblastic) leukemia, acute myelocytic leukemia, myeloid leukemia, chronic lymphocytic leukemia, multiple myeloma, plasma cell leukemia, and chronic myelocytic leukemia; the lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenstrom's macroglobulinemia; the sarcoma is selected from osteosarcoma, Ewing's sarcoma, leiomyosarcoma, synovial sarcoma, soft tissue sarcoma, angiosarcoma, liposarcoma, fibrosarcoma, rhabdomyosarcoma, and chondrosarcoma. Preferably, the tumor is selected from pancreatic cancer, liver cancer, oral squamous cell carcinoma, colon cancer, ovarian cancer, and gastric cancer. In a specific embodiment of the present invention, the tumor is a lymphoma.

"CMV-related disease" described herein includes diseases caused by CMV pp65 infection, including neonatal CMV inclusion body disease, which may severely affect the liver, spleen and central nervous system, as well as disability. Also included are acute acquired CMV infections, similar to infectious mononucleosis, including fever, skeletal muscle pain, and the like. Also included are immunocompromised people, such as those transplanted with an organ, or those with HIV, whose infection may lead to CMV retinitis, gastrointestinal CMV, encephalitis, and the like.

"Antigen-binding fragment" described herein includes, but is not limited to: a Fab fragment, having VL, CL, VH and CH1 domains; a Fab' fragment, which is a Fab fragment having one or more cysteine residues at the C-terminus of the CH1 domain; an Fd fragment, having VH and CH1 domains; an Fd' fragment, having VH and CH1 domains and one or more cysteine residues at the C-terminus of the CH1 domain; an Fv fragment, having VL and VH domains of a single arm of an antibody; a dAb fragment, consisting of a VH domain or a VL domain; an isolated CDR region; an F(ab')₂ fragment, which is a bivalent fragment comprising two Fab' fragments connected by a disulfide bridge at the hinge region; a single chain antibody molecule (e.g., single chain Fv; scFv); a "diabody" with two antigen-binding sites, comprising a heavy chain variable domain (VH) linked to a light chain variable domain (VL) in the same polypeptide chain; a "linear antibody", comprising a pair of tandem Fd segments (VH-CH1-VH-CH1) that, together with a complementary light chain polypeptide, form a pair of antigen-binding regions; and a modified form of any of the foregoing, which retains antigen-binding activity.

"CDR" described herein is a short fragment of an immunoglobulin (Ig) or a T cell antigen receptor (TCR) that binds to an antigen epitope alone or in combination with other CDRs. The immunoglobulin may be an antibody, and the CDRs correspond to complementarity determining regions within the variable sequences of the antibody. For each variable region, there are three CDRs in each variable region of the heavy and light chains, which are referred to as heavy-chain or light-chain CDR1, CDR2 and CDR3, respectively. In the T cell antigen receptor (TCR), the CDRs are present in the α or β chain, and there are three CDRs in each of the α or β chain, which are referred to as α-chain or β-chain CDR1, CDR2 and CDR3, respectively. The exact boundaries of these CDRs are defined differently according to different systems. The system described by Kabat et al. (Kabat et al, Sequences of Proteins of Immunological Interest (National Institutes of Health, Bethesda, Md. (1987) and (1991)) provides not only a clear residue numbering system applicable to antibody variable regions, but also residue boundaries defining the three CDRs. Those CDRs may be referred to as Kabat CDRs. Each complementarity determining region may comprise amino acid residues from a "complementarity determining region" as defined by Kabat. Chothiaet al. (Chothia & Lesk, J. mol. Biol, 196: 901-917 (1987) and Chothiaet al., Nature 342:877-883 (-1989)) found that certain sub-portions within the Kabat CDRs adopts almost identical peptide backbone conformation, although with large diversity at the amino acid sequence level. Those sub-portions are referred to as L1, L2 and L3, or H1, H2 and H3, respectively, where "L" and "H" represent the light and heavy chain regions, respectively. Those regions may be referred to as Chothia CDRs, which have boundaries that overlap with those of Kabat CDRs. There are some other CDRs whose boundaries may not be defined strictly following one of the above systems, but will still overlap with those of the Kabat CDRs. CDRs defined according to any of these systems may be used in the methods used herein, although CDRs defined by Kabat or Chothia are used in preferred embodiments. The residue boundaries of the CDRs in the TCR are as described above. "Antibody variable region" refers to the portion of the light and heavy chains of an antibody molecule that includes the amino acid sequences of the complementarity determining regions (CDRs, i.e., CDR1, CDR2 and CDR3) and the framework regions (FRs). VH refers to the variable domain of the heavy chain. VL refers to the variable domain of the light chain.

When the "comprise" or "comprising" described herein is used to describe a sequence of a protein or nucleic acid in the present application, the protein or nucleic acid may be consisted of the sequence, or may have additional amino acids or nucleotides at one or both ends of the protein or nucleic acid, but still possess the activity described herein.

"Prevent" or "prevention" described herein refers to the suppression of symptoms or the delay of all actions by which a particular symptom is stressed by administration of the product of the present invention.

"Diagnosis" or "diagnosing" described herein refers to the determination of whether a patient has suffered from, is suffering from, or will suffer from a disease or condition in the past, at the time of diagnosis, or in the future, or the determination of the progression or likely progression in the future of a disease, or the assessment of a patient's response to a therapy.

"Treatment" or "treating" described herein refers to slowing, interrupting, arresting, controlling, stopping, alleviating, or reversing the progression or severity of a sign, symptom, disorder, condition or disease, but does not necessarily involve the complete elimination of all disease-related signs, symptoms, conditions or disorders, and refers to therapeutic intervention that ameliorates the signs, symptoms, and the like of a disease or pathological state after the disease has begun to progress.

"Effective amount" described herein refers to an amount or dose of the product described herein which provides the desired treatment or prevention after administration to a patient or organ in single or multiple doses. "Product" described herein includes, but is not limited to the antibody or the antigen-binding fragment thereof, the T cell antigen receptor, the nucleic acid, the expression vector, the host cell, the immune cell or the multimeric complex described herein, and an additional agent that assists or cooperates with the above products. "Product" described herein may be a pharmaceutical composition such as a kit, a chip, an antibody conjugate or a multifunctional antibody.

"Subject" described herein includes, but is not limited to, a human or non-human mammal. Preferably, the non-human mammal includes, but is not limited to, a mouse, rat, monkey, pig or rabbit, or the like.

"Homology" described herein means that in the context of using an amino acid sequence or a nucleotide sequence, those skilled in the art can adjust the sequence according to as necessary for practice without changing the main structure or function of the original sequence, so as to obtain a sequence having (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99% or 100% homology to the specific sequence described herein. For example, "having at least 80% homology to any one of SEQ ID NOs: 12-17" described herein means that while retaining the binding function of the cytomegalovirus (CMV) phosphoprotein pp65 peptide epitope:MHC complex, SEQ ID NO: 12, 13, 14, 15, 16 or 17 can be adjusted as necessary for practice, including one or more alterations, for example, by substitution, deletion and/or insertion of one or more amino acids, or truncations, or lengthening at one or both ends, as long as the sequence retains 80% or more homology and retains the binding function to the pp65 epitope:MHC complex or pp65 epitope:MHC molecule tetramer. The alterations may be substitutions, additions or deletions, and may be substitutions between amino acids of the same polarity, substitutions between amino acids of the same charge, substitutions between uncharged amino acids, substitutions between aliphatic amino acids, substitutions between aromatic amino acids, substitutions between amino acids having no polarity, or substitutions between amino acids having a side chain moiety of a relevant property. The basic side chain includes, but is not limited to, lysine, arginine or histidine. The acidic side chain includes, but is not limited to, aspartic acid or glutamic acid. The uncharged amino acids include, but are not limited to, aspartyl, glutamine, serine, threonine or tyrosine. The nonpolar side chain includes, but is not limited to, glycine, alanine, valine, leucine, isoleucine, proline, phenylalanine, methionine, tryptophan or cysteine. "At least 80%" includes, but is not limited to, 80%, 81%, 82%, 83%, 84%, 85%, 86%, 87%, 88%, 89%, 90%, 91%, 92%, 93%, 94%, 95%, 96%, 97%, 98%, 99% or 100%.

### BRIEF DESCRIPTION OF THE DRAWINGS

Hereinafter, embodiments of the present invention will be described in detail with reference to the accompanying drawings, in which:
FIG. 1: the SDS PAGE detection result of the antigen peptide-MHC monomer, wherein M is a protein Marker, and the strips 1 and 2 are monomers;
FIG. 2: the detection result of the tetramer fishing for specific T cells, wherein the first row is comparison results of the self-developed A0201-NLV tetramer and the commercialized tetramer fishing for specific T cells, and the second row is comparison results of the self-developed A2402-QYD tetramer and the commercialized tetramer fishing for specific T cells;
FIG. 3: the detection of the positive rate of C44 TCR-T cells by the A1101-ATV tetramer;
FIG. 4: a schematic diagram of the linking of TCR β and α chains in a pHAGE vector, wherein the linking is performed by the following order: a promoter, the β chain, furin-p2A, the α chain, IRES and RFP sequences;
FIG. 5: the expression of the membrane surface as assayed by flow cytometry, wherein FIG. 5A shows the expression of the membrane surface of HLA-A^{∗}A0201 NLVPMVATV specific TCRs (C2, C22, C29), FIG. 5B shows the expression of the membrane surface of HLA-A*A2402 QYDPVAALF specific TCRs (C27, C30), and
FIG. 5C shows the expression of the membrane surface of HLA-A^{∗}A1101 ATVQGQNLK specific TCRs (C44, C45), in which BV421 is one of fluoresceins, and BV stands for Brilliant Violet;
FIG. 6: the affinity of TCRs for binding to the CMV pp65 tetramer probe as assayed by flow cytometry, wherein
FIG. 6A shows the detection result of the affinity of HLA-A*A0201 NLVPMVATV specific TCRs (C2, C22, C29) for binding to the CMV pp65 tetramer probe, FIG. 6B shows the detection result of the affinity of HLA-A*A2402 QYDPVAALF specific TCRs (C27, C30) for binding to the CMV pp65 tetramer probe, and FIG. 6C shows the detection result of the affinity of HLA-A*A1101 ATVQGQNLK specific TCRs (C44, C45) for binding to the CMV pp65 tetramer probe;
FIG. 7: the detection results of the release levels of C2-TCR T, C22-TCR T, C29-TCR T cytokines IL2 (FIG. 7B), TNFα (FIG. 7C) and IFNγ (FIG. 7D) and the luciferase level of target cells (FIG. 7A), wherein C2, C22 and C29 are TCRs prepared in Example 2, NE represents a blank control group, NT represents a T cell-only group, Raji is Raji cells untransfected with pp65, and 1G4 represents a specific TCR against NY-ESO-1;
FIG. 8: the detection results of the release levels of C27-TCR T, C30-TCR T cytokines IL2 (FIG. 8B), TNFα (FIG. 8C) and IFNγ (FIG. 8D) and the luciferase level of target cells (FIG. 8A), wherein C27 and C30 are TCRs prepared in Example 2, NE represents a blank control group, NT represents a T cell-only group, Raji is Raji cells untransfected with pp65, and 1G4 represents a specific TCR against NY-ESO-1;
FIG. 9: the detection results of the release levels of C44-TCR T, C45-TCR T cytokines IFNγ (FIG. 9B) and the luciferase level of target cells (FIG. 9A), wherein C44 and C45 are TCRs prepared in Example 2, NE represents a blank control group, NT represents a T cell-only group, Raji is Raji cells untransfected with pp65, and 1G4 represents a specific TCR against NY-ESO-1;
FIG. 10: evaluation of the inhibition of C22-TCR on tumor growth in mice in a lymphoma animal model;
FIG. 11: statistics for the survival rates of mice in a lymphoma animal model, a T cell-free injection group (PBS), a control T-cell injection group (NC), and a CMV TCR-T injection group (C22-TCR);
FIG. 12: statistics for the weight of mice in the lymphoma animal model, the T cell-free injection group (PBS), the control T-cell injection group (NC), and the CMV TCR-T injection group (C22-TCR);
FIG. 13: statistics for TCR T cell-specific proliferation of mice in the lymphoma animal model, the T cell-free injection group (PBS), the control T-cell injection group (NC), and the CMV TCR-T injection group (C22-TCR), which show that the results of the PBS group and the NC group are both 0, and 6 broken lines of FIG. 13 are statistics for TCR T cell-specific proliferation of six different mice in the C22-TCR group;
FIG. 14: evaluation of the inhibition of C27-TCR on tumor growth in mice in a lymphoma animal model;
FIG. 15: statistics for the tumor growth of mice in a lymphoma animal model, a T cell-free injection group (PBS), a control T-cell injection group (NC), and a CMV TCR-T injection group (C27-TCR);
FIG. 16: statistics for TCR T cell-specific proliferation of mice in the lymphoma animal model, the T cell-free injection group (PBS), the control T-cell injection group (NC), and the CMV TCR-T injection group (C27-TCR);
FIG. 17: evaluation of the inhibition of C45-TCR on tumor growth in mice in a lymphoma animal model;
FIG. 18: statistics for the tumor growth of mice in a lymphoma animal model, a T cell-free injection group (PBS), a control T-cell injection group (NC), and a CMV TCR-T injection group (C45-TCR); and
FIG. 19: statistics for TCR T cell-specific proliferation of mice in the lymphoma animal model, the T cell-free injection group (PBS), the control T-cell injection group (NC), and the CMV TCR-T injection group (C45-TCR).

### DETAILED DESCRIPTION

Technical solutions in the embodiments of the present invention will be described clearly and completely below with reference to the drawings. It is apparent that the described embodiments are only a part of the embodiments of the present invention, but not all of them. Based on the embodiments of the present invention, all other embodiments obtained by those of ordinary skill in the art without making creative work shall fall within the protection scope of the present invention.

### Example 1: Construction of and Effect Assay on CMV Antigen Epitope Tetramers

### I. Construction of CMV antigen epitope tetramers

1) An α chain and a β2m chain (with the amino acid sequence set forth in SEQ ID NO: 54, and the nucleotide sequence set forth in SEQ ID NO: 55) of HLA-A*0201 (with the amino acid sequence set forth in SEQ ID NO: 48, and the nucleotide sequence set forth in SEQ ID NO: 49), HLA-A*2402 (with the amino acid sequence set forth in SEQ ID NO: 50, and the nucleotide sequence set forth in SEQ ID NO: 51) and HLA-A^{∗}1101 (with the amino acid sequence set forth in SEQ ID NO: 52, and the nucleotide sequence set forth in SEQ ID NO: 53) with optimized expression sequences were provided. The structure of the α chain was as follows: the extracellular domain sequence of the α chain of the corresponding HLA type was connected with an Avi-tag sequence, with a BamHI enzyme cutting site as a spacer to provide a biotinylation site. The β2m chain was depleted of the signal peptide sequence, with two amino acids (M and A) added before a mature peptide sequence. The expression vector was PET28a+, and the expression strain was transetta or BL21. IPTG was at a concentration of 0.5 mM, and the expression was induced for 4 h. The protein inclusion bodies of the α chain and β2m chain were extracted.
2) Selection of CMV antigen epitopes (antigen peptides): HLA-A*0201 type corresponds to an antigen epitope NLVPMVATV (SEQ ID NO: 1), HLA-A*2402 type corresponds to an antigen epitope QYDPVAALF (SEQ ID NO: 2), and HLA-A^{∗}1101 type corresponds to an antigen epitope ATVQGQNLK (SEQ ID NO: 3).
3) Folding and purification of pMHC I monomer: the antigen peptides in the step 2), and the corresponding β2m chain renaturation proteins and the α chain proteins in the step 1) were added into a reduction system in order according to the molar ratio of 40:2:1, and the folding reaction was performed for 72 h. The resulting products were purified on a Superdex75 10/300GL column. The purified products were collected, biotinylated using an avidity kit, and purified again to obtain biotinylated monomers, which were determined for the purity by gel electrophoresis.
4) The biotinylated monomers in the step 3) were subjected to a binding reaction with APC-labeled streptavidin to obtain corresponding tetramers, which were named as A0201-NLVPMVATV-tetramer (A0201-NLV tetramer for short), A2402-QYDPVAALF-tetramer (A2402-QYD tetramer for short) and A1101-ATVQGQNLK-tetramer (A1101-ATV tetramer for short).

### II. Assay on the effect of CMV antigen epitope tetramers

1. Human peripheral blood mononuclear cells (PBMCs) were isolated or TCR-T cells were prepared, and a cell suspension was prepared at a cell density of 1×10⁶ cells/mL.
2. The cells were centrifuged at 3000 rpm for 5 min. The supernatant was removed and resuspended in 50 µL of PBS containing 1% serum.
3. 2 µL of tetramer was added, and the mixture was incubated at room temperature for 30 min.
4. 2 µL of CD8 antibody was added, and the mixture was incubated on ice for 20 min.
5. 1 mL of PBS was added, and the mixture was centrifuged at 3000 rpm for 5 min.
6. The supernatant was removed, 1 mL of PBS was added, and the mixture was centrifuged at 3000 rpm for 5 min.
7. The supernatant was removed, the cells were resuspended in 500 µL of 4% paraformaldehyde, and the cell suspension was filtered through a filter membrane.
8. Positive cells were detected by a flow cytometer.

### III. Experimental results

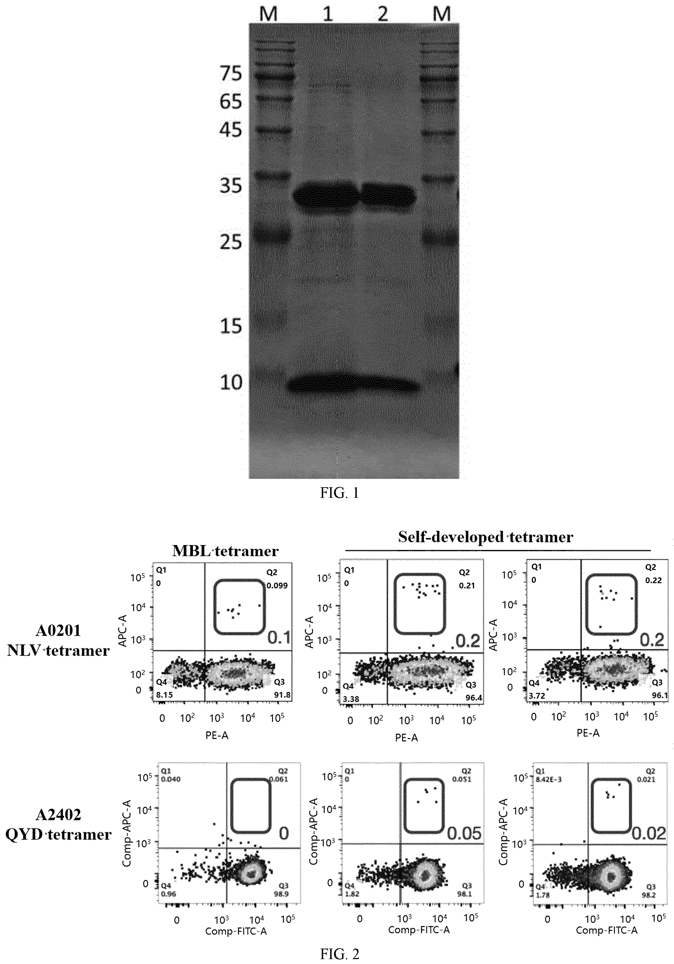

The SDS PAGE detection results of the monomers are shown in FIG. 1. As shown in FIG. 1, after refolding and HPLC purification, for the resulting monomers, the proteins with sizes corresponding to those of the heavy chain (the α-chain extracellular domain connected with the Avi-tag sequence at the C-terminus) and the light chain (the β2m chain depleted of the signal peptide region), respectively, and having higher purity are clearly showed. The constructed tetramers were separately co-incubated with corresponding HLA type PBMC cells to fish for specific T cells. As shown in FIG. 2, the self-developed tetramer has a significant advantage over the commercial tetramer (from MBL) in fishing for specific T cells. Specifically, the self-developed A0201-NLV tetramer sorted specific T cells twice as many as the commercial tetramer (MBL, TS-0010-2C). In addition, the sorting efficiency of different batches was similar, thereby showing extremely high sorting efficiency and stability. The A0201-NLV tetramer was used to fish for C22 TCR and C29 TCR. The commercial A2402-QYD (MBL, TS-0020-2C) tetramer did not detect corresponding specific T cells, while the self-developed tetramer detected 0.02%-0.05% of the positive cell population, which indicated that the self-developed tetramer was highly sensitive in sorting specific T cells and the data from different batches showed high stability. The A2402-QYD tetramer was used to fish for C27 TCR and C30 TCR.

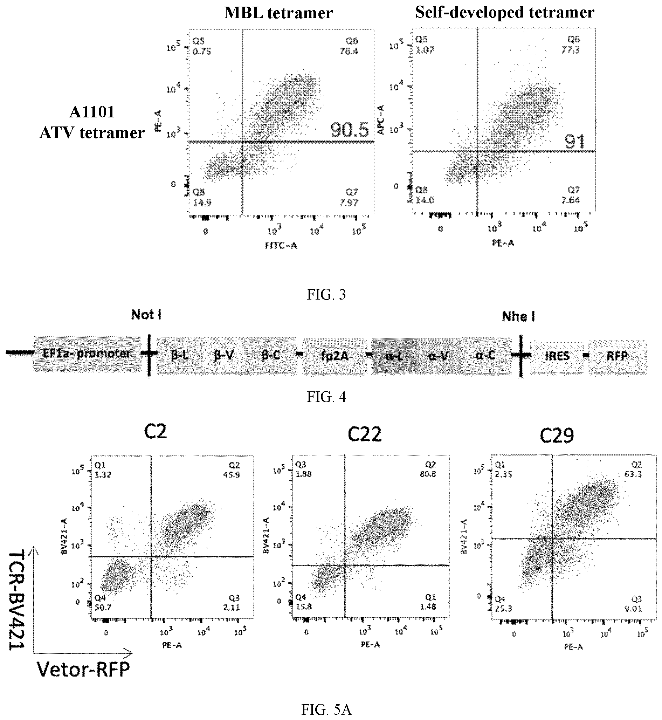

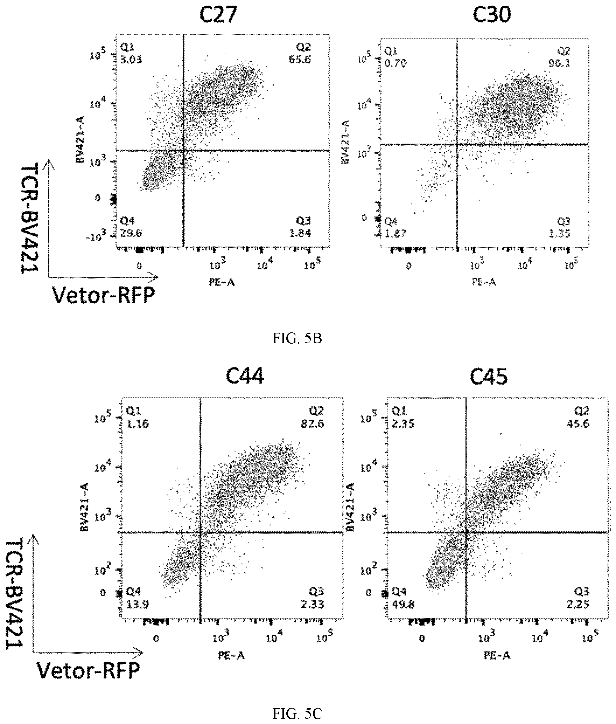

Meanwhile, the TCR corresponding to A1101 type (C44 TCR) could be simultaneously recognized by the self-developed tetramer and the commercialized tetramer on the same epitope (MBL, TS-M012-1) (see FIG. 3), and the positive cell population in the commercial tetramer accounted for 90.5% of the infected cells, and that of the self-developed tetramer accounted for 91% of the infected cells. The results for C45 TCR are shown in FIG. 5.

### Example 2: Construction of pHAGE-TCR-RFP Vector

### I. Acquisition of β and α gene fragments of CMV pp65 epitope-specific TCRs

1) The HLA-A*0201-NLVPMVATV-tetramer, HLA-A*2402- QYDPVAALF-tetramer and HLA-A*1101-ATVQGQNLK-tetramer which were purchased from MBL or self-prepared were stained with peripheral blood according to the product instructions, T cells positive for tetramer staining were sorted by flow cytometry and subjected to reverse transcription to obtain cDNA (SuperScript^{®} IV Reverse Transcriptase, Invitrogen). The variable region fragments of the TCRβ gene were obtained by amplification by two rounds of PCR (KOD-Plus-Neo, TOYOBO) based on the principle of multiplex PCR.

Reverse transcription primer: TRBC1-TCAGGCAGTATCTGGAGTCATTG (SEQ ID NO: 136)

PCR amplification primers:
Upstream primer 1: T cell receptor β variable region-TRBV_F1 (see SEQ ID NOs: 56-95)
Upstream primer 2: T cell receptor β variable region-TRBV_F2 (see SEQ ID NOs: 96-135)
Downstream primer 1: T cell receptor β constant region-TRBC2-GCACCTCCTTCCCATTCACC (SEQ ID NO: 137)
Downstream primer 2: T cell receptor β constant region-TRBC3-GCTTCTGATGGCTCAAACACAG (SEQ ID NO: 138)

Specifically, according to the product instructions of the PCR polymerase KOD-Plus-Neo, the PCR system of the first round was at 20 µL, the annealing temperature was 60 °C, and the reaction was performed for 30 cycles. 1 µL of the product from the first round of PCR reaction was taken as a template of the second round of PCR, wherein the PCR system of the second round was at 30 µL, the annealing temperature was 60 °C, and the reaction was performed for 30 cycles. The product from the second round of PCR was subjected to agarose gel electrophoresis, and the band with the corresponding size was extracted from gel (TIANGEN Gel Extraction Kit) and sent for sequencing, wherein the sequencing primer was the downstream primer 2. The TCRβ gene sequences were obtained. The β-chain nucleotide sequences of C2, C22, C27, C29, C30, C44 and C45 are set forth in SEQ ID NOs: 277, 260-265.

2) As above, reverse transcription was performed on T cells positive for tetramer staining to obtain cDNA (SuperScript^{®} IV Reverse Transcriptase, Invitrogen). The TCRα gene fragments were obtained by amplification by two rounds of PCR (KOD-Plus-Neo, TOYOBO) according to the product instructions.
Reverse transcription primer: T cell receptor α constant region-TRAC1-CGACCAGCTTGACATCACAG (SEQ ID NO: 139)

PCR amplification primers:
Upstream primer 3: T cell receptor α variable region-TRAV_F1 (see SEQ ID NOs: 142-186)
Upstream primer 4: T cell receptor α variable region-TRAV_F2 (see SEQ ID NOs: 187-228)
Downstream primer 3: T cell receptor α constant region-TRAC2-GTTGCTCTTGAAGTCCATAGACCTC (SEQ ID NO: 140)
Downstream primer 4: T cell receptor α constant region-TRAC3-CAGGGTCAGGGTTCTGGATA (SEQ ID NO: 141)

Specifically, according to the product instructions of the PCR polymerase KOD-Plus-Neo, the PCR system of the first round was at 20 µL, the annealing temperature was 60 °C, and the reaction was performed for 30 cycles. 1 µL of the product from the first round of PCR reaction was taken as a template of the second round of PCR, wherein the PCR system of the second round was at 30 µL, the annealing temperature was 60 °C, and the reaction was performed for 30 cycles. The product from the second round of PCR was subjected to agarose gel electrophoresis, and the band with the corresponding size was extracted from gel (TIANGEN Gel Extraction Kit) and sent for sequencing, wherein the sequencing primer was the downstream primer 4. The TCRα gene sequences were obtained. Specifically, the α-chain nucleotide sequences of C2, C22, C27, C29, C30, C44 and C45 are set forth in SEQ ID NOs: 278, 266-271.

### II. Construction of pHAGE-TCR vector

TCRβ, fp2A and TCRα were amplified by overlap-PCR (KOD-Plus-Neo, TOYOBO) with long primer (containing fp2A sequence) to obtain TCRβ-fp2A-TCRα fragments, which were named as C22, C27, C29, C30, C44 or C45, respectively.

The primers for amplification included an upstream primer 5 (Table 1), a downstream primer 5, an upstream primer 6 (Table 2), and a downstream primer 6.

**Table 1**

| TCR | Upstream primer 5 |
|---|---|
| C2 | atttcaggtgtcgtgaagcggccgcgccaccATGGGCCCCCAGCTCCT (SEQ ID NO: 279) |
| C22 | atttcaggtgtcgtgaagcggccgcgccaccATGAGCATCGGCCTCCT (SEQ ID NO: 229) |
| C27 | atttcaggtgtcgtgaagcggccgcgccaccATGAGCATCGGCCTCCT (SEQ ID NO: 230) |
| C29 | atttcaggtgtcgtgaagcggccgcgccaccATGGACTCCTGGACCTT (SEQ ID NO: 231) |
| C30 | atttcaggtgtcgtgaagcggccgcgccaccATGGGCACCAGGCTCCT (SEQ ID NO: 232) |
| C44 | atttcaggtgtcgtgaagcggccgcgccaccATGGGCCCCGGGCTCCT (SEQ ID NO: 233) |
| C45 | atttcaggtgtcgtgaagcggccgcgccaccATGGGCTCCAGGCTGCT (SEQ ID NO: 234) |

Downstream primer 5:

TCTCCAGCCTGCTTCAGCAGGCTGAAGTTAGTAGCTCCGCTTCCGCTccgtttccgccgGAAATCCTTTCT
CTTGACCATG (SEQ ID NO: 235)

**Table 2**

| TCR | Upstream primer 6 |
|---|---|
| C2 | TCAGCCTGCTGAAGCAGGCTGGAGACGTGGAGGAGAACCCTGGACCT ATGATGGAGAAGAATCCTTTGGCA (SEQ ID NO: 280) |
| C22 | TCAGCCTGCTGAAGCAGGCTGGAGACGTGGAGGAGAACCCTGGACCTATGGAGAAGAATCCTTTGGCA GCC (SEQ ID NO: 236) |
| C27 | TCAGCCTGCTGAAGCAGGCTGGAGACGTGGAGGAGAACCCTGGACCTATGGAGAAGAATCCTTTGGCA GCC (SEQ ID NO: 237) |
| C29 | TCAGCCTGCTGAAGCAGGCTGGAGACGTGGAGGAGAACCCTGGACCTATGATGAAATCCTTGAGAGTT TTA (SEQ ID NO: 238) |
| C30 | TCAGCCTGCTGAAGCAGGCTGGAGACGTGGAGGAGAACCCTGGACCTATGGAGAAGAATCCTTTGGCA GCC (SEQ ID NO: 239) |
| C44 | TCAGCCTGCTGAAGCAGGCTGGAGACGTGGAGGAGAACCCTGGACCTATGGCATGCCCTGGCTTCCTG TGG (SEQ ID NO: 240) |
| C45 | TCAGCCTGCTGAAGCAGGCTGGAGACGTGGAGGAGAACCCTGGACCTATGGCCATGCTCCTGGGGGCA TCA (SEQ ID NO: 241) |

Downstream primer 6: agggatcctctagactcgagctagcTCAGCTGGACCACAGCCGCA (SEQ ID NO: 242).

Specifically, the TCRβ and the TCRα were firstly obtained by amplification by using a primer 5 and a primer 6, respectively, wherein the PCR system was at 50 µL, the annealing temperature was 60 °C, and the reaction was performed for 30 cycles. The PCR products were subjected to gel electrophoresis and extracted (TIANGEN Gel Extraction Kit), and the extracted products were taken as templates, each at 1 µL, and subjected to overlap PCR by using an upstream primer 5 and a downstream primer 6, respectively, wherein the PCR system was at 50 µL, the annealing temperature was 60 °C, and the reaction was performed for 30 cycles. The product was subjected to agarose gel electrophoresis to obtain a band of about 1800bp, which was then extracted from gel.

The lentiviral vector pHAGE-IRES-RFP was double digested with NotI and NheI, wherein the enzyme digestion system was at 40 µL, wherein the NotI and NheI were each at 1.5µL, the plasmid was at 2-3 µg, and the enzyme digestion was performed at 37 °C for 6 h. Then 1 µL of alkaline phosphatase (NEB) was added into the system and treated for 1 h to reduce the self-ligation of the plasmid, and the plasmid after the enzyme digestion was subjected to gel electrophoresis and extracted, determined for the concentration using nanodrop, and used as a backbone for constructing the plasmid.

According to the product instructions of Clone Express II One Step Cloning kit, the TCR was connected with a linearized pHAGE-IRES-RFP vector after enzyme digestion through overlap (see FIG. 4), and transformed into Stbl3 strain, which was then cultured in an ampicillin-containing LB plate for 12-16 h. The monoclonal strain was picked for sequencing, wherein the sequencing primers selected were primers seq-pHAGE-F and seq-pHAGE-R on the pHAGE vector and a downstream primer 4. Corresponding TCRs were obtained, abbreviated as C22 (with the nucleotide sequence set forth in SEQ ID NO: 37, and the amino acid sequence set forth in SEQ ID NO: 36), C27 (with the nucleotide sequence set forth in SEQ ID NO: 39, and the amino acid sequence set forth in SEQ ID NO: 38), C29 (with the nucleotide sequence set forth in SEQ ID NO: 41, and the amino acid sequence set forth in SEQ ID NO: 40), C30 (with the nucleotide sequence set forth in SEQ ID NO: 43, and the amino acid sequence set forth in SEQ ID NO: 42), C44 (with the nucleotide sequence set forth in SEQ ID NO: 45, and the amino acid sequence set forth in SEQ ID NO: 44), C45 (with the nucleotide sequence set forth in SEQ ID NO: 47, and the amino acid sequence set forth in SEQ ID NO: 46) and C2 (with the nucleotide sequence set forth in SEQ ID NO: 274, the amino acid sequence set forth in SEQ ID NO: 273, the amino acid sequence of the β chain set forth in SEQ ID NO: 275, and the amino acid sequence of the α chain set forth in SEQ ID NO: 276).

Wherein, C2 was prepared as a control, which differed from the CDR regions of C22 by only a few amino acids, and the details are shown in Table 3.

**Table 3**

| NO. | CDR1α | CDR2α | CDR3α | CDR1β | CDR2β | CDR3β |
|---|---|---|---|---|---|---|
| C2 | SSNFYA (SEQ ID NO: 4) | MTLNGDE (SEQ ID NO: 8) | ASPYFNKFY (SEQ ID NO: 281) | MNHEY (SEQ ID NO: 18) | SMNVEV (SEQ ID NO: 282) | ASSPTLGTGAETQY (SEQ ID NO: 283) |
| C22 | SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |

### Example 3: Assay on Membrane Expression and Affinity of TCRs by a pMHC Tetramer Staining Method

### 1. Construction of endogenous TCR knockout Jurkat T cell lines

Based on the sequence characteristics of the Jurkat cell TCR, guide sequences (TRA_oligol-CACCGTCTCTCAGCTGGTACACGGC (SEQ ID NO: 243), TRA_oligo2-AAACGCCGTGTACCAGCTGAGAGAC (SEQ ID NO: 244), TRB_oligol-CACCGGGCTCAAACACAGCGACCTC (SEQ ID NO: 245), TRB_oligo2-AAACGAGGTCGCTGTGTTTGAGCCC (SEQ ID NO: 246)) were designed in the constant regions of the α chain and the β chain.

The synthesized guide sequences of the α chain and the β chain were constructed into sgRNA-LentiCRISPR-puro and sgRNA-LentiCRISPR-BSD lentiviral vectors, respectively, and the vectors were co-transfected with packaging plasmids psPAX2 and pMD2.G and a PEI transfection reagent into 293T cells according to a certain ratio. The cell culture supernatants were harvested at 48 h and 72 h and concentrated, and the two viruses after concentration were simultaneously used to infect a human Jurkat T cell line. 48 h after the infection, killing was performed using puromycin and blasticidin at appropriate concentrations until all cells in the control group for each of the two drugs were dead. Surviving cells were sorted by flow cytometry to obtain single cells, which were added into a 96-well plate for culturing. For the obtained monoclonal cell line, its expression was separately identified using antibodies of the TCRα chain and the TCRβ chain, and the cell strain defective in both chains was the obtained endogenous TCR knockout Jurkat T cell, which was named as JC5.

### Construction of JC5 cell line stably integrating CMV TCR

The pHAGE-TCR plasmids such as C22, C27, C29, C30, C44 or C45 constructed in Example 2 were separately mixed with packaging plasmids psPAX2 and pMD2.G and a PEI transfection reagent according to a certain ratio, and transfected into 293T cells. The cell culture supernatants were harvested at 48 h and 72 h and concentrated to infect JC5 cells in the logarithmic growth phase (MOI = 0.3). 3 days after infection, cells were stained with anti-human CD3 and anti-human TCRαβ flow cytometry antibodies, and the cells with the same TCR expression level were sorted and cultured to obtain the JC5-TCR cell line.

### 3. Assay on expression-on-membrane and affinity of TCRs

1×10⁶ JC5-TCR cells were taken, stained with Brilliant Violet 421^{™} anti-human TCRαβ (Biolegend) and the corresponding CMV pp65pMHC tetramer-APC (tetramer-APC) and then analyzed by flow cytometry.

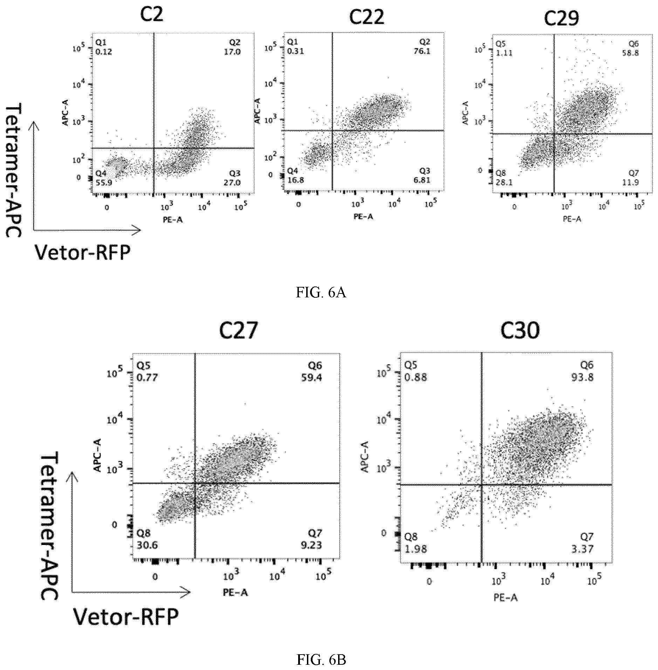

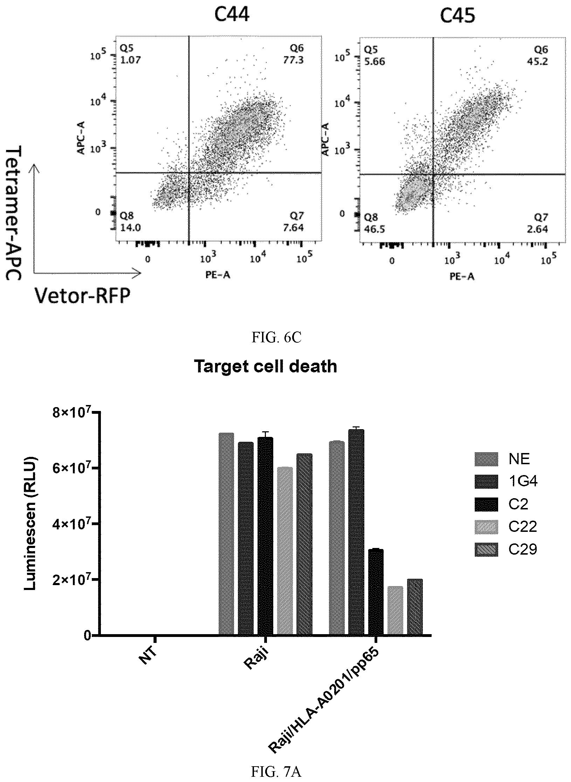

As can be seen from FIGs. 5 and 6, the prepared specific C22-TCR and C29-TCR against CMV pp65 HLA-A*A0201 NLVPMVATV were both able to be correctly expressed and displayed on the outer side of the cell membrane, and had certain affinity for the corresponding tetramer probe. The C2-TCR was compared to the C22-TCR in that the CDR regions differed by only a few amino acids, and the differences in the effect of the expression-on-membrane (FIG. 5A) and the affinity for the corresponding tetramer probe (FIG. 6A) were significant. The specific C27-TCR and C30-TCR against CMV pp65 HLA-A*A2402 QYDPVAALF and the specific C44-TCR and C45-TCR against CMV pp65 HLA-A*A1101 ATVQGQNLK were all correctly expressed and displayed on the outer side of the cell membrane and had a certain affinity for the corresponding tetramer probe. In summary, the results show that the TCR prepared in the example of the present application has good affinity.

### Example 4: Construction of and In Vitro Functional Assay on Human Primary TCR T Cells

### 1. Isolation, culture and lentivirus infection of human primary T cells

To further verify the recognition and killing function of the selected TCRs for the CMV pp65 antigens, mononuclear cells (PBMCs) were isolated from peripheral blood of volunteers using the lymphocyte isolation solution Ficoll, then T cells were obtained from PBMCs by negative selection according to the product instructions of EasySep Human T cell isolation kit (stem cell technologies), resuspended to 1×10⁶ cells/mL in a 1640 complete medium containing 100 U/mL IL2, and cultured in an anti-CD3/CD28 antibody-coated culture dish for activation. After 48 h of activation, the T cells were infected with the TCR-loaded viral particles (prepared in Example 3) using a lentivirus system by centrifuging at 1500 rpm for 2 h at 32 °C, culturing in a 37 °C cell incubator for 10 h and terminating the infection by media exchange, and then cultured in a 37 °C cell incubator. Three days after infection, TCR positive cells were sorted by flow cytometry to obtain TCRT cells (including C2, C22, C27, C29, C30, C44 and C45 as described above).

### 2. Construction of target cells

Virus particles separately loaded with pp65-PURO, HLA-A*0201-BSD, HLA-A*2402-BSD, HLA-A*1101-BSD and luciferase-GFP were used to infect into Raji cells in the logarithmic growth phase using a lentivirus system. Raji cells simultaneously stably expressing pp65, HLA-A*0201/2402/1101 molecules and luciferase-GFP were obtained by drug screening and flow cytometry sorting, and named as Raji-A0201-pp65-luciferase, Raji-A2402-pp65-luciferase and Raji-A1101-pp65-luciferase, respectively.

### 3. In vitro functional verification of TCRs in human primary T cells

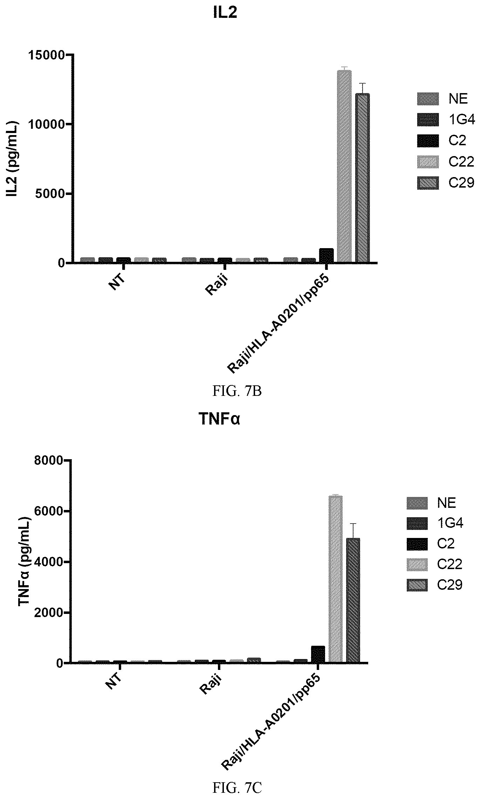

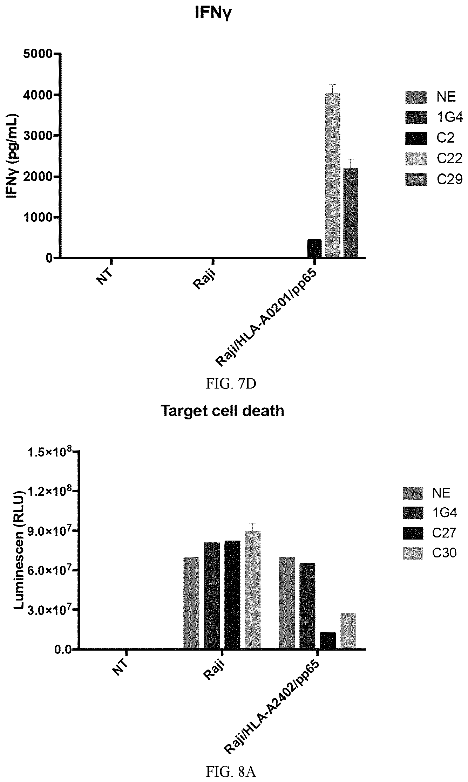

Raji/HLA-A0201/pp65 and Raji cells untransfected with pp65 (named as Raji) were each separately co-incubated with 1G4-TCRT cells, TCR-C2T cells, TCR-C22T cells and TCR-C29T cells according to a quantity ratio of 1:1. After 24 h of co-incubation, the cells and supernatant were separately collected, and the activation of TCR-C22T cells and TCR-C29T cells and the death of target cells were preliminarily determined. In terms of the release levels of the extracellular cytokines TNFα, IL2 and IFNγ (see FIG. 7), TCR-C22T and TCR-C29T, when co-incubated with target cells, were able to significantly cause the activation of T cells as compared to the control groups 1G4-TCRT and TCR-C2T. The amount of luciferase released from the target cells after lysis may reflect the death of the target cells (see FIG. 7), and TCR-C22T and TCR-C29T prepared in the example of the present application have a significant killing effect on target cells compared to TCR-C2T with similar CDR structure. Experimental results show that the TCR-C22T cells and TCR-C29T cells constructed in the example of the present invention can be specifically activated by CMV pp65 antigen peptide presenting cells, and can significantly kill target cells.

Meanwhile, the hypervariable region of CDR3 of the α chain and the β chain of the TCRs has the greatest effect on TCR function. The experiment has proved that the hypervariable region of CDR3, even if highly similar with only one amino acid difference, was functionally far apart. For example, the amino acids after the first position of CDR3 regions of the α chain and the β chain of TCR E141 were mutated into alanine in sequence and constructed onto a lentiviral vector to prepare corresponding TCR-T cells. The TCR-T cells were co-cultured with target cells at a ratio of 1:1, and the amount of luciferase and the secreted amount of cytokines TNFα, IL2 and IFNγ were determined. The results show that the unmutated E141, when exposed to HLAmatched-Raji, can effectively clear target cells and specifically produce a large amount of cytokine IL2. In addition, mutations of a single amino acid at the sites a3, a5, a6 and b6, b7, b8 are sufficient to completely inactivate TCR T cells (see Patent CN202010373100.4 for details). Accordingly, the mutated TCR, although differing by only one amino acid, is sufficient to completely inactivate the TCR-T cells. Therefore, although the target is the same, only one amino acid difference may produce a completely different technical effect for two nearly identical TCRs, let alone for multiple amino acids.

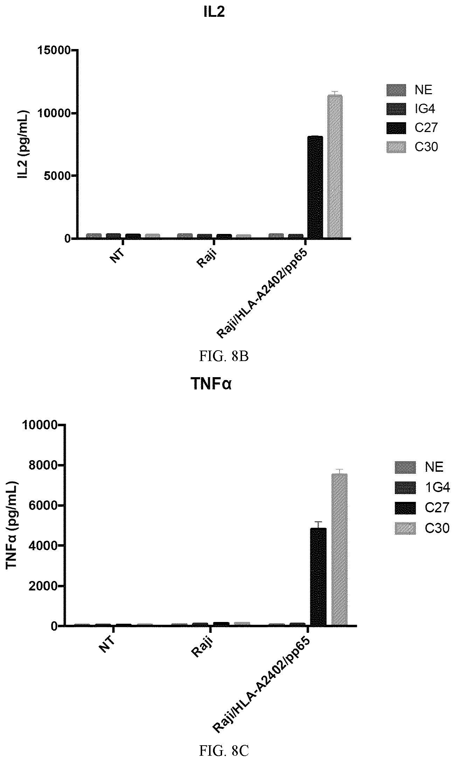

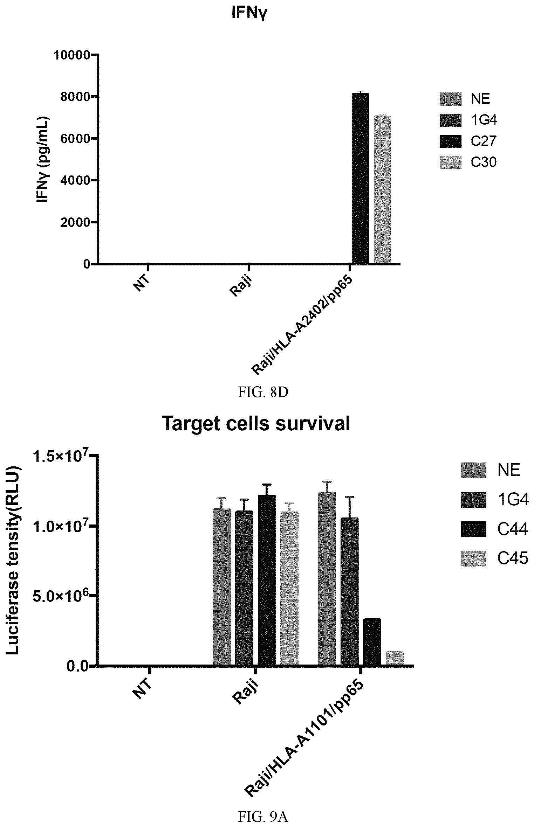

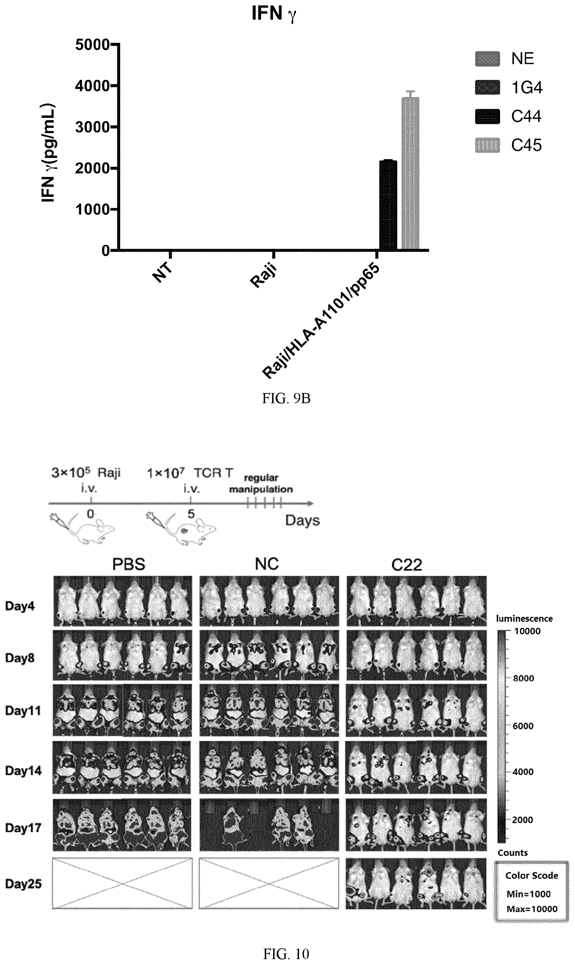

Raji/HLA2402/pp65 and Raji cells untransfected with pp65 (named as Raji) were each separately co-incubated with 1G4-TCRT cells, TCR-C27T cells and TCR-C30T cells according to a quantity ratio of 1:1. After 24 h of co-incubation, the cells and supernatant were separately collected, and the activation of TCR-C27T cells and TCR-C30T cells and the death of target cells were preliminarily determined. In terms of the release levels of the extracellular cytokines TNFα, IL2 and IFNγ (see FIG. 8), TCR-C27T cells and TCR-C30T cells, when co-incubated with target cells, were able to significantly cause the activation of T cells as compared to the control group 1G4-TCRT. Moreover, the amount of luciferase released from the target cells after lysis may reflect that TCR-C27T and TCR-C30T can significantly kill target cells (see FIG. 8). Experimental results show that the TCR-C27T cells and TCR-C30T cells constructed in the example of the present invention can be specifically activated by CMV pp65 antigen peptide presenting cells, and can significantly kill target cells. Raji/HLA1101/pp65 and Raji cells untransfected with pp65 (named as Raji) were each separately co-incubated with 1G4-TCRT cells, TCR-C44T cells and TCR-C45T cells according to a quantity ratio of 1:1. After 24 h of co-incubation, the cells and supernatant were separately collected, and the activation of TCR-C44T cells and TCR-C45T cells and the death of target cells were preliminarily determined. In terms of the release levels of the extracellular cytokine IFNγ (see FIG. 9), TCR-C44T cells and TCR-C45T cells, when co-incubated with target cells, were able to significantly cause the activation of T cells as compared to the control group 1G4-TCRT. Moreover, the amount of luciferase released from the target cells after lysis may reflect that TCR-C44T and TCR-C45T can significantly kill target cells (see FIG. 9). Experimental results show that the TCR-C44T cells and TCR-C45T cells constructed in the example of the present invention can be specifically activated by CMV pp65 antigen peptide presenting cells, and can significantly kill target cells.

### Example 5: Animal Model Construction and In Vivo Functional Assay on CMV TCR-T

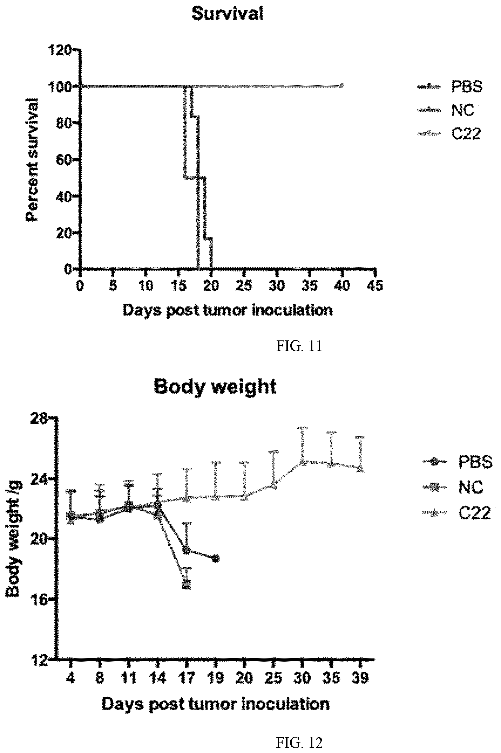

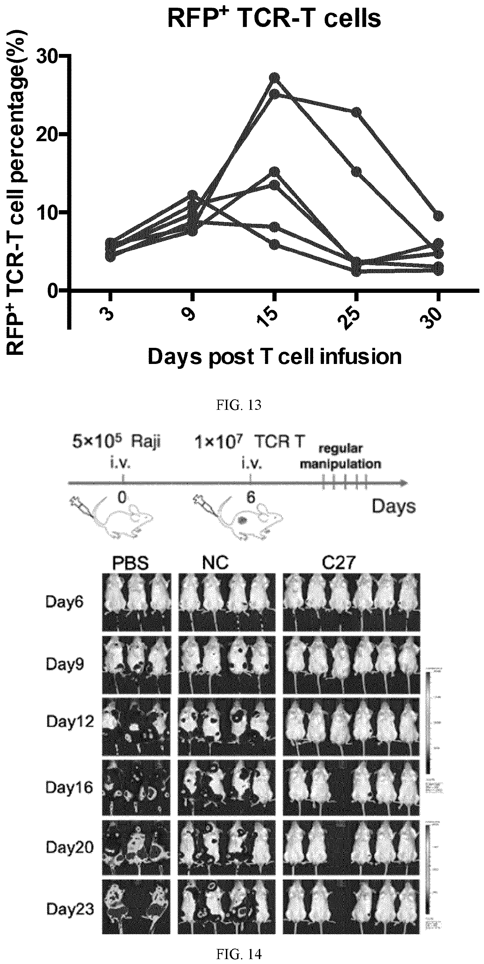

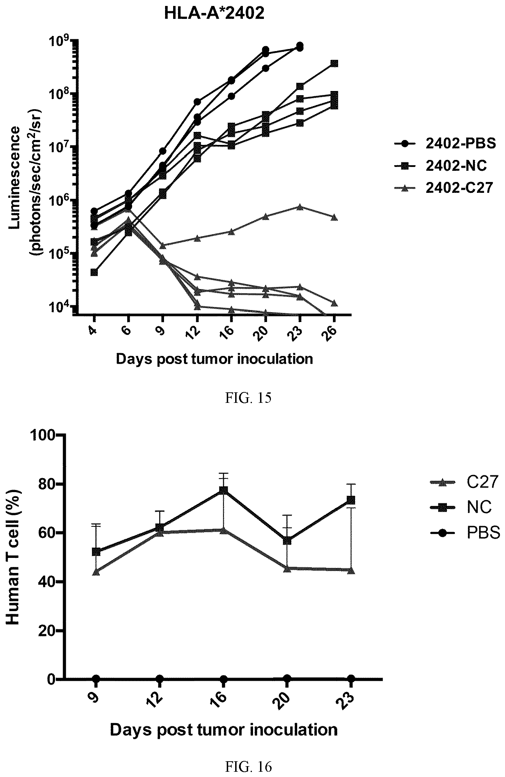

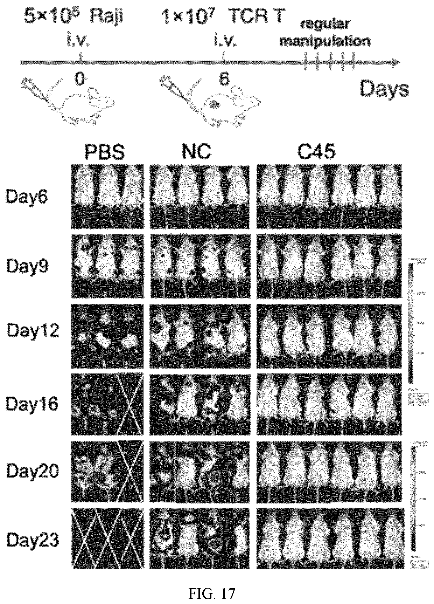

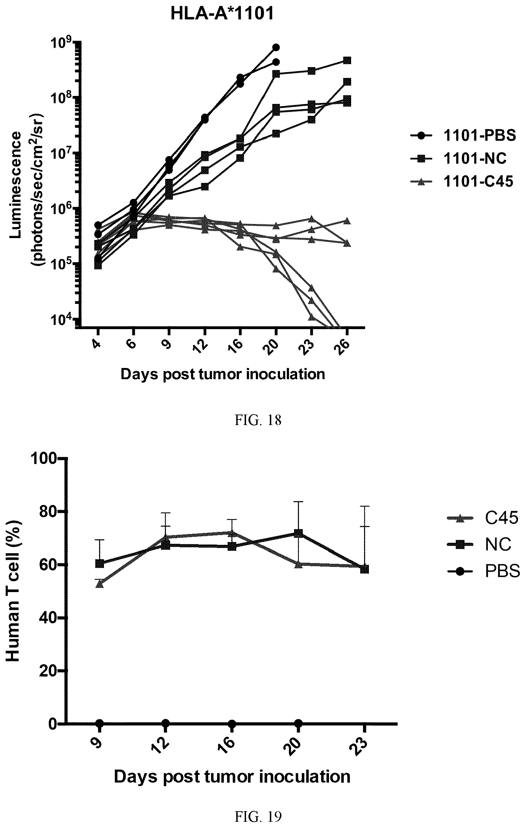

NOD/Scid IL-2Ry null (NCG) female mice aged 5-6 weeks were inoculated with 3×10⁵ Raji-HLA-A*1101/0201/2402-pp65-luciferase tumor cells via tail veins to construct a lymphoma model (see FIGs. 10, 14 and 17), which was recorded as day 1. On day 6, the mice were divided into 3 groups, i.e., A: a PBS injection group (with equal volume of PBS injected); B: a control NC cell injection group (NC T cells); and C: a CMV TCR T injection group (C22/C27/C45-TCR T cells), wherein the mice in group B were injected with 1×10⁷ NC T cells via tail veins, the mice in group C were injected with 1×10⁷ TCR T cells via tail veins, and the mice in group A were injected with equal volume (200 µL) of PBS. Specific reinfusion volume and reinfusion time points for other TCR T cells are shown in FIGs 14 and 17. The mice were monitored for tumor cell growth, T cell proliferation and mouse survival over the next few weeks. As shown in FIGs. 10-13, compared with the control group, the CMV-specific C22-TCR T cells constructed in the example of the present invention were able to significantly kill tumor cells in mice (FIG. 10) and increase the survival rate of mice (FIG. 11, FIG. 12), and the returned TCR T cells proliferated specifically *in vivo* (FIG. 13). In addition, as shown in FIGs. 14-19, compared with the control group, the CMV-specific C27-TCR T, C45-TCR T cells constructed in the example of the present invention were also able to significantly kill tumor cells in mice.

The preferred embodiments of the present invention are described in detail above, which, however, are not intended to limit the present invention. Within the scope of the technical concept of the present invention, various simple modifications can be made to the technical solution of the present invention, all of which will fall within the protection scope of the present invention.

In addition, it should be noted that the various specific technical features described in the above specific embodiments can be combined in any suitable manner without contradiction. In order to avoid unnecessary repetition, such combinations will not be illustrated separately.

## Claims

1. A complementarity determining region (CDR) binding to CMV pp65, wherein the CDR is selected from one of or a combination of two or more of SEQ ID NOs: 4-33.

2. The CDR according to claim 1, comprising CDR1α-CDR3α and/or CDR1β-CDR3β, wherein the CDR1α has an amino acid sequence comprising any one of SEQ ID NOs: 4-7 or having at least 80% homology to any one of SEQ ID NOs: 4-7, the CDR2α has an amino acid sequence comprising any one of SEQ ID NOs: 8-11 or having at least 80% homology to any one of SEQ ID NOs: 8-11, the CDR3α has an amino acid sequence comprising any one of SEQ ID NOs: 12-17 or having at least 80% homology to any one of SEQ ID NOs: 12-17, the CDR1β has an amino acid sequence comprising any one of SEQ ID NOs: 18-22 or having at least 80% homology to any one of SEQ ID NOs: 18-22, the CDR2β has an amino acid sequence comprising any one of SEQ ID NOs: 23-27 or having at least 80% homology to any one of SEQ ID NOs: 23-27, and the CDR3β has an amino acid sequence comprising any one of SEQ ID NOs: 28-33 or having at least 80% homology to any one of SEQ ID NOs: 28-33.

3. The CDR according to claim 1 or 2, selected from any one of the following groups:
| CDR1α | CDR2α | CDR3α | CDR1β | CDR2β | CDR3β |
|---|---|---|---|---|---|
| SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |
| SSNFYA (SEQ ID NO: 4) | MTLNGDE (SEQ ID NO: 8) | ASGTYKYI (SEQ ID NO: 13) | MNHEY (SEQ ID NO: 18) | SVGAGI (SEQ ID NO: 23) | ASRPLGVGETQY (SEQ ID NO: 29) |
| NSAFQY (SEQ ID NO: 5) | TYSSGN (SEQ ID NO: 9) | ATFLTGNQFY (SEQ ID NO: 14) | SGHDY (SEQ ID NO: 19) | FNNNVP (SEQ ID NO: 24) | ASSFSLPYEQY (SEQ ID NO: 30) |
| SSNFYA (SEQ ID NO: 4) | MTLNGDE (SEQ ID NO: 8) | ARYGNKLV (SEQ ID NO: 15) | SGHVS (SEQ ID NO: 20) | FQNEAQ (SEQ ID NO: 25) | ASSFYTGQETQY (SEQ ID NO: 31) |
| TSESDYY (SEQ ID NO: 6) | QEAYKQQN (SEQ ID NO: 10) | AYRAFYTGANSKLT (SEQ ID NO: 16) | SGHDT (SEQ ID NO: 21) | YYEEEE (SEQ ID NO: 26) | ASSFFRGEGNQPQH (SEQ ID NO: 32) |
| NSMFDY (SEQ ID NO: 7) | ISSIKDK (SEQ ID NO: 11) | AASAWNTGNQFY (SEQ ID NO: 17) | SGHRS (SEQ ID NO: 22) | YFSETQ (SEQ ID NO: 27) | ASSLAGYKQETQY (SEQ ID NO: 33) |

4. An α-chain polypeptide binding to CMV pp65, wherein the α-chain polypeptide comprises a CDR1α, a CDR2α and/or a CDR3α, wherein the CDR1α has an amino acid sequence comprising any one of SEQ ID NOs: 4-7 or having at least 80% homology to any one of SEQ ID NOs: 4-7, the CDR2α has an amino acid sequence comprising any one of SEQ ID NOs: 8-11 or having at least 80% homology to any one of SEQ ID NOs: 8-11, and the CDR3α has an amino acid sequence comprising any one of SEQ ID NOs: 12-17 or having at least 80% homology to any one of SEQ ID NOs: 12-17.

5. The α-chain polypeptide according to claim 4, comprising CDR1α-CDR3α of any one of the following groups:
| CDR1α | CDR2α | CDR3α |
|---|---|---|
| SSNFYA (SEQ ID NO: 4) | MTLNGDE (SEQ ID NO: 8) | ARNTGNQFY (SEQ ID NO: 12) |
| SSNFYA | MTLNGDE | ASGTYKYI |
| (SEQ ID NO: 4) | (SEQ ID NO: 8) | (SEQ ID NO: 13) |
| NSAFQY (SEQ ID NO: 5) | TYSSGN (SEQ ID NO: 9) | ATFLTGNQFY (SEQ ID NO: 14) |
| SSNFYA (SEQ ID NO: 4) | MTLNGDE (SEQ ID NO: 8) | ARYGNKLV (SEQ ID NO: 15) |
| TSESDYY (SEQ ID NO: 6) | QEAYKQQN (SEQ ID NO: 10) | AYRAFYTGANSKLT (SEQ ID NO: 16) |
| NSMFDY (SEQ ID NO: 7) | ISSIKDK (SEQ ID NO: 11) | AASAWNTGNQFY (SEQ ID NO: 17) |

6. A β-chain polypeptide binding to CMV pp65, wherein the β-chain polypeptide comprises a CDR1β, a CDR2β and/or a CDR3β, wherein the CDR1β has an amino acid sequence comprising any one of SEQ ID NOs: 18-22 or having at least 80% homology to any one of SEQ ID NOs: 18-22, the CDR2β has an amino acid sequence comprising any one of SEQ ID NOs: 23-27 or having at least 80% homology to any one of SEQ ID NOs: 23-27, and the CDR3β has an amino acid sequence comprising any one of SEQ ID NOs: 28-33 or having at least 80% homology to any one of SEQ ID NOs: 28-33.

7. The β-chain polypeptide according to claim 6, comprising CDR1β-CDR3β of any one of the following groups:
| | | |
|---|---|---|
| CDR1β | CDR2β | CDR3β |
| MNHEY (SEQ ID NO: 18) | SVGAGI (SEQ ID NO: 23) | ASSFATGVGSYGYT (SEQ ID NO: 28) |
| MNHEY (SEQ ID NO: 18) | SVGAGI (SEQ ID NO: 23) | ASRPLGVGETQY (SEQ ID NO: 29) |
| SGHDY (SEQ ID NO: 19) | FNNNVP (SEQ ID NO: 24) | ASSFSLPYEQY(SEQ ID NO: 30) |
| SGHVS (SEQ ID NO: 20) | FQNEAQ (SEQ ID NO: 25) | ASSFYTGQETQY (SEQ ID NO: 31) |
| SGHDT (SEQ ID NO: 21) | YYEEEE (SEQ ID NO: 26) | ASSFFRGEGNQPQH (SEQ ID NO: 32) |
| SGHRS (SEQ ID NO: 22) | YFSETQ (SEQ ID NO: 27) | ASSLAGYKQETQY (SEQ ID NO: 33) |

8. An antibody or an antigen-binding fragment thereof, wherein the antibody or the antigen-binding fragment thereof specifically binding to CMV pp65, comprising CDR H1-CDR H3 of a heavy chain and/or CDR L1-CDR L3 of a light chain, wherein the CDR H3 has an amino acid sequence comprising any one of SEQ ID NOs: 12-17 or having at least 80% homology to any one of SEQ ID NOs: 12-17, and the CDR L3 has an amino acid sequence comprising any one of SEQ ID NOs: 28-33 or having at least 80% homology to any one of SEQ ID NOs: 28-33.

9. The antibody or the antigen-binding fragment thereof according to claim 8, wherein the CDR H1 has an amino acid sequence comprising any one of SEQ ID NOs: 4-7 or having at least 80% homology to any one of SEQ ID NOs: 4-7, the CDR H2 has an amino acid sequence comprising any one of SEQ ID NOs: 8-11 or having at least 80% homology to any one of SEQ ID NOs: 8-11, the CDR L1 has an amino acid sequence comprising any one of SEQ ID NOs: 18-22 or having at least 80% homology to any one of SEQ ID NOs: 18-22, and the CDR L2 has an amino acid sequence comprising any one of SEQ ID NOs: 23-27 or having at least 80% homology to any one of SEQ ID NOs: 23-27.

10. The antibody or the antigen-binding fragment thereof according to claim 8 or 9, wherein the CMV pp65 has a binding epitope comprising any one of or a combination of two or three of SEQ ID NOs: 1-3.

11. The antibody or the antigen-binding fragment thereof according to any one of claims 8 to 10, wherein the CDR H1-CDR H3 and the CDR L1-CDR L3 are selected from any one of the following groups:
| | Binding epitope | CDR H1 | CDR H2 | CDR H3 | CDR L1 | CDR L2 | CDR L3 |
|---|---|---|---|---|---|---|---|
| C22 | SEQ ID NO: 1 | SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |
| C27 | SEQ ID NO: 2 | SSNFYA (SEQ ID NO: 4) | MTLNGDE (SEQ ID NO: 8) | ASGTYKYI (SEQ ID NO: 13) | MNHEY (SEQ ID NO: 18) | SVGAGI (SEQ ID NO: 23) | ASRPLGVGETQY (SEQ ID NO: 29) |
| C29 | SEQ ID NO: 1 | NSAFQY(SEQ ID NO: 5) | TYSSGN (SEQ ID NO: 9) | ATFLTGNQFY (SEQ ID NO: 14) | SGHDY (SEQ ID NO: 19) | FNNNVP (SEQ ID NO: 24) | ASSFSLPYEQY(SEQ ID NO: 30) |
| C30 | SEQ ID NO: 2 | SSNFYA(SEQ ID NO: 4) | MTLNGDE (SEQ ID NO: 8) | ARYGNKLV (SEQ ID NO: 15) | SGHVS (SEQ ID NO: 20) | FQNEAQ (SEQ ID NO: 25) | ASSFYTGQETQY (SEQ ID NO: 31) |
| C44 | SEQ ID NO: 3 | TSESDYY (SEQ ID NO: 6) | QEAYKQQN (SEQ ID NO: 10) | AYRAFYTGANSKLT (SEQ ID NO: 16) | SGHDT (SEQ ID NO: 21) | YYEEEE (SEQ ID NO: 26) | ASSFFRGEGNQPQH (SEQ ID NO: 32) |
| C45 | SEQ ID NO: 3 | NSMFDY (SEQ ID NO: 7) | ISSIKDK (SEQ ID NO: 11) | AASAWNTGNQFY (SEQ ID NO: 17) | SGHRS (SEQ ID NO: 22) | YFSETQ (SEQ ID NO: 27) | ASSLAGYKQETQY (SEQ ID NO: 33) |

12. The antibody or the antigen-binding fragment thereof according to any one of claims 8 to 11, wherein the light chain has an amino acid sequence comprising any one of SEQ ID NOs: 247-252 or having at least 80% homology to any one of SEQ ID NOs: 247-252.

13. The antibody or the antigen-binding fragment thereof according to any one of claims 8 to 12, wherein the heavy chain has an amino acid sequence comprising any one of SEQ ID NOs: 253-258 or having at least 80% homology to any one of SEQ ID NOs: 253-258.

14. The antibody or the antigen-binding fragment thereof according to any one of claims 8 to 13, wherein the antibody or the antigen-binding fragment thereof is a single domain antibody or a single chain antibody scFv.

15. The antibody or the antigen-binding fragment thereof according to any one of claims 8 to 14, wherein the antibody or the antigen-binding fragment thereof has an amino acid sequence comprising any one of SEQ ID NOs: 36, 38, 40, 42, 44 or 46 or having at least 80% homology to any one of SEQ ID NOs: 36, 38, 40, 42, 44 or 46.

16. A T cell antigen receptor, wherein the T cell antigen receptor specifically binding to CMV pp65, wherein the T cell antigen receptor comprises CDR1α-CDR3α of an α chain and/or CDR1β-CDR3β of a β chain, wherein the CDR3α has an amino acid sequence comprising any one of SEQ ID NOs: 12-17 or having at least 80% homology to any one of SEQ ID NOs: 12-17, and the CDR3β has an amino acid sequence comprising any one of SEQ ID NOs: 28-33 or having at least 80% homology to any one of SEQ ID NOs: 28-33.

17. The T cell antigen receptor according to claim 16, wherein the CDR1α has an amino acid sequence comprising any one of SEQ ID NOs: 4-7 or having at least 80% homology to any one of SEQ ID NOs: 4-7, the CDR2α has an amino acid sequence comprising any one of SEQ ID NOs: 8-11 or having at least 80% homology to any one of SEQ ID NOs: 8-11, the CDR1β has an amino acid sequence comprising any one of SEQ ID NOs: 18-22 or having at least 80% homology to any one of SEQ ID NOs: 18-22, and the CDR2β has an amino acid sequence comprising any one of SEQ ID NOs: 23-27 or having at least 80% homology to any one of SEQ ID NOs: 23-27.

18. The T cell antigen receptor according to any one of claims 16 to 17, wherein the CMV pp65 has a binding epitope comprising any one of or a combination of two or three of SEQ ID NOs: 1-3.

19. The T cell antigen receptor according to any one of claims 16 to 18, wherein the CDR1α-CDR3α and the CDR1β-CDR3β comprise any one of the following groups:
| TCR | Binding epitope | CDR1α | CDR2α | CDR3α | CDR1β | CDR2β | CDR3β |
|---|---|---|---|---|---|---|---|
| C22 | SEQ ID NO: 1 | SSNFYA (SEQ ID NO: 4) | MTLNGDE (SEQ ID NO: 8) | ARNTGNQFY (SEQ ID NO: 12) | MNHEY (SEQ ID NO: 18) | SVGAGI (SEQ ID NO: 23) | ASSFATGVGSYGYT (SEQ ID NO: 28) |
| C27 | SEQ ID NO: 2 | SSNFYA (SEQ ID NO: 4) | MTLNGDE (SEQ ID NO: 8) | ASGTYKYI (SEQ ID NO: 13) | MNHEY (SEQ ID NO: 18) | SVGAGI (SEQ ID NO: 23) | ASRPLGVGETQY (SEQ ID NO: 29) |
| C29 | SEQ ID NO: 1 | NSAFQY(SEQ ID NO: 5) | TYSSGN (SEQ ID NO: 9) | ATFLTGNQFY (SEQ ID NO: 14) | SGHDY (SEQ ID NO: 19) | FNNNVP (SEQ ID NO: 24) | ASSFSLPYEQY(SEQ ID NO: 30) |
| C30 | SEQ ID NO: 2 | SSNFYA(SEQ ID NO: 4) | MTLNGDE (SEQ ID NO: 8) | ARYGNKLV (SEQ ID NO: 15) | SGHVS (SEQ ID NO: 20) | FQNEAQ (SEQ ID NO: 25) | ASSFYTGQETQY (SEQ ID NO: 31) |
| C44 | SEQ ID NO: 3 | TSESDYY (SEQ ID NO: 6) | QEAYKQQN (SEQ ID NO: 10) | AYRAFYTGANSKLT (SEQ ID NO: 16) | SGHDT (SEQ ID NO: 21) | YYEEEE (SEQ ID NO: 26) | ASSFFRGEGNQPQH (SEQ ID NO: 32) |
| C45 | SEQ ID NO: 3 | NSMFDY (SEQ ID NO: 7) | ISSIKDK (SEQ ID NO: 11) | AASAWNTGNQFY (SEQ ID NO: 17) | SGHRS (SEQ ID NO: 22) | YFSETQ (SEQ ID NO: 27) | ASSLAGYKQETQY (SEQ ID NO: 33) |

20. The T cell antigen receptor according to any one of claims 16 to 19, wherein the β chain has an amino acid sequence comprising any one of SEQ ID NOs: 247-252 or having at least 80% homology to any one of SEQ ID NOs: 247-252.

21. The T cell antigen receptor according to any one of claims 16 to 20, wherein the α chain has an amino acid sequence comprising any one of SEQ ID NOs: 253-258 or having at least 80% homology to any one of SEQ ID NOs: 253-258.

22. The T cell antigen receptor according to any one of claims 16 to 21, wherein amino acids of the α chain and the β chain are linked directly or indirectly, preferably linked indirectly, and more preferably via fp2A.

23. The T cell antigen receptor according to any one of claims 16 to 22, wherein the T cell antigen receptor has an amino acid sequence comprising any one of SEQ ID NOs: 36, 38, 40, 42, 44 or 46 or having at least 80% homology to any one of SEQ ID NOs: 36, 38, 40, 42, 44 or 46.

24. A nucleic acid encoding the CDR according to any one of claims 1 to 3.

25. A nucleic acid encoding the β chain of the T cell antigen receptor according to any one of claims 16 to 23 or the β-chain polypeptide according to any one of claims 6 to 7.

26. The nucleic acid according to claim 25, wherein the nucleic acid has a sequence comprising any one of SEQ ID NOs: 260-265 or having at least 80% homology to any one of SEQ ID NOs: 260-265.

27. A nucleic acid encoding the α chain of the T cell antigen receptor according to any one of claims 16 to 23 or the α-chain polypeptide according to any one of claims 4 to 5.

28. The nucleic acid according to claim 27, wherein the nucleic acid has a sequence comprising any one of SEQ ID NOs: 266-271 or having at least 80% homology to any one of SEQ ID NOs: 266-271.

29. A nucleic acid encoding the T cell antigen receptor according to any one of claims 16 to 23 or the antibody or the antigen-binding fragment thereof according to any one of claims 8 to 15.

30. The nucleic acid according to claim 29, wherein the nucleic acid has a sequence comprising any one of SEQ ID NOs: 37, 39, 41, 43, 45 or 47 or having at least 80% homology to any one of SEQ ID NOs: 37, 39, 41, 43, 45 or 47.

31. An expression vector, wherein the expression vector comprises the nucleic acid according to any one of claims 24 to 30.

32. A host cell, wherein the host cell comprises the nucleic acid according to any one of claims 24 to 30 or the expression vector according to claim 31.

33. An immune cell, wherein the immune cell expresses the CDR according to any one of claims 1 to 3, the α-chain polypeptide according to any one of claims 4 to 5, the β-chain polypeptide according to any one of claims 6 to 7, the antibody or the antigen-binding fragment thereof according to any one of claims 8 to 15, or the T cell antigen receptor according to any one of claims 16 to 23.

34. The immune cell according to claim 33, wherein the immune cell comprises one or more of the heterologous nucleic acid sequences according to any one of claims 24 to 30.

35. The immune cell according to claim 33 or 34, wherein the immune cell is selected from a T cell and a stem cell.

36. The immune cell according to any one of claims 33 to 35, wherein the immune cell is isolated from a T cell derived from a subject.

37. A method for preparing an immune cell, wherein the method comprises transfecting an immune cell with a nucleic acid sequence encoding the CDR according to any one of claims 1 to 3, the α-chain polypeptide according to any one of claims 4 to 5, the β-chain polypeptide according to any one of claims 6 to 7, the antibody or the antigen-binding fragment thereof according to any one of claims 8 to 15, or the T cell antigen receptor according to any one of claims 16 to 23 for expression.

38. The method according to claim 37, further comprising a step of knocking out an endogenous TCR of the cell.

39. A method for preparing a recombinant T cell, comprising the following steps:
1) obtaining the nucleic acid according to any one of claims 24 to 30 from a positive T cell clone;
2) separating, culturing a primary T cell; and
3) delivering the nucleic acid obtained in the step 1) to the primary T cell in the step 2) to obtain a recombinant T cell expressing the CDR according to any one of claims 1 to 3, the α-chain polypeptide according to any one of claims 4 to 5, the β-chain polypeptide according to any one of claims 6 to 7, the antibody or the antigen-binding fragment thereof according to any one of claims 8 to 15, or the T cell antigen receptor according to any one of claims 16 to 23.

40. A method for preparing a T cell antigen receptor, comprising the following steps:
(1) obtaining the nucleic acid according to any one of claims 24 to 30 from a positive T cell clone;
(2) connecting the nucleic acid obtained in the step (1) to a vector backbone to obtain an expression vector;
(3) transforming the expression vector obtained in the step (2) into a host cell, then inducing the expression of the host cell; and
(4) obtaining a T cell antigen receptor.

41. A multimeric complex, comprising the T cell antigen receptor according to any one of claims 16 to 23.

42. The multimeric complex according to claim 41, further comprising a monomer, a biotin molecule, and a streptavidin or avidin molecule, wherein the monomer comprises an α-chain extracellular domain of an MHC molecule, a β2m chain and an antigen peptide, and the monomer is conjugated to the biotin molecule binding to the streptavidin or avidin molecule.

43. The multimeric complex according to claim 42, wherein the antigen peptide comprises any one of or a combination of two or more of SEQ ID NOs: 1-3.

44. The multimeric complex according to claim 42 or 43, wherein the MHC molecule is selected from HLA-A*0201, HLA-A*2402 and HLA-A*1101.

45. A method for preparing the multimeric complex according to any one of claims 41 to 44, comprising the following steps:
I) expressing and purifying an α-chain extracellular domain of an MHC molecule connected with an avi-tag sequence at the C terminus and a β2m chain;
II) refolding an antigen peptide, the β2m chain obtained in the step 1), and the α-chain extracellular domain of the MHC molecule connected with the avi-tag sequence at the C terminus to prepare a monomer;
III) biotinylating the monomer prepared in the step II) to obtain a biotinylated monomer;
IV) subjecting the biotinylated monomer obtained in the step III) to a reaction with fluorescently labeled streptavidin or avidin to prepare an antigen peptide-MHC molecule tetramer; and
V) co-incubating the antigen peptide-MHC molecule tetramer obtained in the step IV) with T cells to form a complex of a T cell antigen receptor and the antigen peptide-MHC molecule tetramer.

46. Use of the CDR according to any one of claims 1 to 3, the α-chain polypeptide according to any one of claims 4 to 5, the β-chain polypeptide according to any one of claims 6 to 7, the antibody or the antigen-binding fragment thereof according to any one of claims 8 to 15, the T cell antigen receptor according to any one of claims 16 to 23, the nucleic acid according to any one of claims 24 to 30, the expression vector according to claim 31, the host cell according to claim 32, the immune cell according to any one of claims 33 to 36, or the multimeric complex according to any one of claims 41 to 44 in preparing a product for the diagnosis or treatment of a tumor or a CMV-related disease.

47. Use of the CDR according to any one of claims 1 to 3, the α-chain polypeptide according to any one of claims 4 to 5, the β-chain polypeptide according to any one of claims 6 to 7, the antibody or the antigen-binding fragment thereof according to any one of claims 8 to 15, the T cell antigen receptor according to any one of claims 16 to 23, the nucleic acid according to any one of claims 24 to 30, the expression vector according to claim 31, the host cell according to claim 32, the immune cell according to any one of claims 33 to 36, or the multimeric complex according to any one of claims 41 to 44 in labeling, detection, cell sorting, or activation of T cells.

48. A pharmaceutical composition, comprising any one of the following groups:
i) the CDR according to any one of claims 1 to 3;
ii) the α-chain polypeptide according to any one of claims 4 to 5;
iii) the β-chain polypeptide according to any one of claims 6 to 7;
iv) the antibody or the antigen-binding fragment thereof according to any one of claims 8 to 15;
v) the T cell antigen receptor according to any one of claims 16 to 23;
vi) the nucleic acid according to any one of claims 24 to 30;
vii) the expression vector according to claim 31;
viii) the host cell according to claim 32;
ix) the immune cell according to any one of claims 33 to 36; or
x) the multimeric complex according to any one of claims 41 to 44.

49. A kit, comprising any one of the following groups:
i) the CDR according to any one of claims 1 to 3;
ii) the α-chain polypeptide according to any one of claims 4 to 5;
iii) the β-chain polypeptide according to any one of claims 6 to 7;
iv) the antibody or the antigen-binding fragment thereof according to any one of claims 8 to 15;
v) the T cell antigen receptor according to any one of claims 16 to 23;
vi) the nucleic acid according to any one of claims 24 to 30;
vii) the expression vector according to claim 31;
viii) the host cell according to claim 32;
ix) the immune cell according to any one of claims 33 to 36; or
x) the multimeric complex according to any one of claims 41 to 44.
